(19) Europäisches Patentamt
European Patent Office
Office européen des brevets

(11) **EP 3 858 989 A1**

(12) **EUROPEAN PATENT APPLICATION**
published in accordance with Art. 153(4) EPC

(43) Date of publication:
**04.08.2021 Bulletin 2021/31**

(51) Int Cl.:
*C12N 9/16* [(2006.01)]        *C12N 15/10* [(2006.01)]
*C12N 15/55* [(2006.01)]       *C12Q 1/6876* [(2018.01)]

(21) Application number: **19866255.3**

(22) Date of filing: **25.09.2019**

(86) International application number:
**PCT/JP2019/037520**

(87) International publication number:
**WO 2020/067122 (02.04.2020 Gazette 2020/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.09.2018  JP 2018179561**

(71) Applicant: **Toray Industries, Inc.
Tokyo, 103-8666 (JP)**

(72) Inventors:
• **UEDA, Yoji
Kamakura-shi, Kanagawa 248-8555 (JP)**
• **TAKII, Yuki
Kamakura-shi, Kanagawa 248-8555 (JP)**
• **ITO, Masateru
Kamakura-shi, Kanagawa 248-8555 (JP)**
• **YAGI, Mai
Kamakura-shi, Kanagawa 248-8555 (JP)**

(74) Representative: **Kador & Partner PartG mbB
Corneliusstraße 15
80469 München (DE)**

(54) **ALKALINE PHOSPHATASE COMPOSITION AND PRODUCTION METHODS FOR DEPHOSPHORYLATED NUCLEIC ACID AND LABELLED NUCLEIC ACID**

(57)    An object of the present invention is to provide a composition containing an alkaline phosphatase and having a high quality, a method for producing a dephosphorylated nucleic acid by using the composition and a method for producing a labeled nucleic acid by using the composition, and in order to achieve the object, the present invention provides a composition containing: an alkaline phosphatase; and a peptide fragment group (A) composed of two or more peptide fragments, wherein each of the two or more peptide fragments consists of 5 to 50 consecutive amino acid residues selected from positions 501 to 578 of the amino acid sequence set forth in SEQ ID NO: 5, wherein a content ratio of the peptide fragment group (A) to the alkaline phosphatase satisfies the following formula (A):

$$(X_A/Y) \times 100 \leq 4.4000 \qquad (A),$$

wherein $X_A$ represents a peak area value of the peptide fragment group (A) calculated by an automatic integration method from an extracted ion chromatogram obtained by an LC-MS/MS analysis of the composition, and Y represents a peak area value of the alkaline phosphatase calculated by an automatic integration method from a chromatogram obtained by an LC-UV analysis of the composition.

EP 3 858 989 A1

SEQUENCE LISTING

<110> TORAY INDUSTRIES, INC.

<120> A composition containing Alkaline Phosphatase

<130> 224017

<160> 5

<170> PatentIn version 3.5

<210> 1
<211> 16
<212> PRT
<213> Bos taurus

<400> 1

Val Pro Leu Ala Ser Glu Thr His Gly Gly Glu Asp Val Ala Val Phe
1               5                   10                  15

<210> 2
<211> 13
<212> PRT
<213> Bos taurus

<400> 2

Val Pro Leu Ala Ser Glu Thr His Gly Gly Glu Asp Val
1               5                   10

<210> 3
<211> 18
<212> PRT
<213> Bos taurus

<400> 3

Gly Pro Gln Ala His Leu Val His Gly Val Gln Glu Glu Thr Phe Val
1               5                   10                  15

Ala His

<210> 4
<211> 12
<212> PRT
<213> Bos taurus

<400> 4

Gly Pro Gln Ala His Leu Val His Gly Val Gln Glu
1               5                   10

<210> 5
<211> 578
<212> PRT
<213> Bos taurus

<400> 5

Met Arg Phe Pro Ser Ile Phe Thr Ala Val Leu Phe Ala Ala Ser Ser
1               5                   10                  15

```
Ala Leu Ala Ala Pro Val Asn Thr Thr Thr Glu Asp Glu Thr Ala Gln
            20              25              30

Ile Pro Ala Glu Ala Val Ile Gly Tyr Ser Asp Leu Glu Gly Asp Phe
        35              40              45

Asp Val Ala Val Leu Pro Phe Ser Asn Ser Thr Asn Asn Gly Leu Leu
    50              55              60

Phe Ile Asn Thr Thr Ile Ala Ser Ile Ala Ala Lys Glu Glu Gly Val
65              70              75              80

Ser Leu Glu Lys Arg Glu Ala Glu Ala Glu Phe Leu Ile Pro Ala Glu
            85              90              95

Glu Glu Asn Pro Ala Phe Trp Asn Arg Gln Ala Ala Gln Ala Leu Asp
            100             105             110

Val Ala Lys Lys Leu Gln Pro Ile Gln Thr Ala Ala Lys Asn Val Ile
        115             120             125

Leu Phe Leu Gly Asp Gly Met Gly Val Pro Thr Val Thr Ala Thr Arg
    130             135             140

Ile Leu Lys Gly Gln Met Asn Gly Lys Leu Gly Pro Glu Thr Pro Leu
145             150             155             160

Ala Met Asp Gln Phe Pro Tyr Val Ala Leu Ser Lys Thr Tyr Asn Val
            165             170             175

Asp Arg Gln Val Pro Asp Ser Ala Gly Thr Ala Thr Ala Tyr Leu Cys
            180             185             190

Gly Val Lys Gly Asn Tyr Arg Thr Asn Gly Lys Leu Gly Pro Glu Thr
        195             200             205

Pro Leu Ala Met Asp Gln Phe Pro Tyr Val Ala Leu Ser Lys Thr Tyr
    210             215             220

Asn Val Asp Arg Gln Val Pro Asp Ser Ala Gly Thr Ala Thr Ala Tyr
225             230             235             240

Leu Cys Gly Val Lys Gly Asn Tyr Arg Thr Tyr Ala His Thr Val Asn
            245             250             255

Arg Asn Trp Tyr Ser Asp Ala Asp Leu Pro Ala Asp Ala Gln Lys Asn
        260             265             270

Gly Cys Gln Asp Ile Ala Ala Gln Leu Val Tyr Asn Met Asp Ile Asp
        275             280             285

Val Ile Leu Gly Gly Gly Arg Met Tyr Met Phe Pro Glu Gly Thr Pro
    290             295             300

Asp Pro Glu Tyr Pro Asp Asp Ala Ser Val Asn Gly Val Arg Lys Asp
305             310             315             320

Lys Gln Asn Leu Val Gln Glu Trp Gln Ala Lys His Gln Gly Ala Gln
        325             330             335

Tyr Val Trp Asn Arg Thr Ala Leu Leu Gln Ala Ala Asp Asp Ser Ser
        340             345             350
```

35

```
Val Thr His Leu Met Gly Leu Phe Glu Pro Ala Asp Met Lys Tyr Asn
        355             360             365

Val Gln Gln Asp His Thr Lys Asp Pro Thr Leu Ala Glu Met Thr Glu
    370             375             380

Ala Ala Leu Gln Val Leu Ser Arg Asn Pro Arg Gly Phe Tyr Leu Phe
385             390             395             400

Val Glu Gly Gly Arg Ile Asp His Gly His His Asp Gly Lys Ala Tyr
            405             410             415

Met Ala Leu Thr Glu Ala Ile Met Phe Asp Asn Ala Ile Ala Lys Ala
            420             425             430

Asn Glu Leu Thr Ser Glu Leu Asp Thr Leu Ile Leu Val Thr Ala Asp
            435             440             445

His Ser His Val Phe Ser Phe Gly Gly Tyr Thr Leu Arg Gly Thr Ser
    450             455             460

Ile Phe Gly Leu Ala Pro Gly Lys Ala Leu Asp Ser Lys Ser Tyr Thr
465             470             475             480

Ser Ile Leu Tyr Gly Asn Gly Pro Gly Tyr Ala Leu Gly Gly Gly Ser
            485             490             495

Arg Pro Asp Val Asn Gly Ser Thr Ser Glu Glu Pro Ser Tyr Arg Gln
            500             505             510

Gln Ala Ala Val Pro Leu Ala Ser Glu Thr His Gly Gly Glu Asp Val
    515             520             525

Ala Val Phe Ala Arg Gly Pro Gln Ala His Leu Val His Gly Val Gln
    530             535             540

Glu Glu Thr Phe Val Ala His Ile Met Ala Phe Ala Gly Cys Val Glu
545             550             555             560

Pro Tyr Thr Asp Cys Asn Leu Pro Ala Pro Ala Thr Ala Thr Ser Ile
            565             570             575

Pro Asp
```

**Description**

BACKGROUND OF THE INVENTION

Field of the Invention

The present invention relates to a composition containing an alkaline phosphatase, a method for producing a dephosphorylated nucleic acid by using the composition and a method for producing a labeled nucleic acid by using the composition.

Background Art

An alkaline phosphatase has a catalyst function that hydrolyzes phosphoric monoesters, and has been widely used in methods for measuring the amount of biological substances such as proteins and nucleic acids (e.g., the immunostaining method, ELISA, the nucleic acid microarray method, etc.). For example, in the research field of genetic engineering, for pretreatment of labeling of nucleic acids such as DNA and RNA and prevention of self-ligation of vectors, dephosphorylation of the 5' end and/or the 3' end of a nucleic acid with an alkaline phosphatase has been performed.

As an industrial production method of an alkaline phosphatase, a production method in which bovine small intestine or large intestine is mainly used as a raw material has been widely adopted since the specific activity of the produced alkaline phosphatase is high. The specific activity of an alkaline phosphatase is generally evaluated by measuring the absorbance at 405 nm derived from p-nitrophenol produced when p-nitrophenylphosphate is decomposed.

A quality of an alkaline phosphatase has been evaluated based on the alkaline phosphatase specific activity. In order to obtain an alkaline phosphatase having a higher specific activity than that of an alkaline phosphatase derived from bovine intestine, an alkaline phosphatase having a high specific activity has been isolated in a purification process or has been produced by using recombinant *Escherichia coli* obtained by a genetic engineering method.

Patent Document 1 discloses a method for producing an alkaline phosphatase having a high specific activity by using recombinant *Escherichia coli* into which an alkaline phosphatase-encoding gene derived from the genus *Bacillus badius* has been introduced. Patent Document 2 discloses a method for producing an alkaline phosphatase having a high specific activity and heat resistance by using recombinant Escherichia coli into which an alkaline phosphatase-encoding gene derived from the genus *Shewanella* has been introduced.

Prior Art Document

[Patent Document]

     Patent Document 1: JP H10-262674 A
     Patent Document 2: WO 2012/115023

SUMMARY OF THE INVENTION

A dephosphorylation reagent containing an alkaline phosphatase (e.g., a commercially available alkaline phosphatase product) is a composition containing other components in addition to the alkaline phosphatase. A quality of a dephosphorylation reagent containing an alkaline phosphatase is evaluated based on the alkaline phosphatase specific activity.

However, it has been found that, even if labeled nucleic acids prepared by using dephosphorylation reagents having almost the same alkaline phosphatase specific activity (labeled nucleic acids obtained by dephosphorylating the 5' ends and/or the 3' ends of nucleic acids with the dephosphorylation reagents, and then binding labeling substances to the 5' ends and/or the 3' ends of the dephosphorylated nucleic acids) are used for a nucleic acid detection method, a great difference in the detection sensitivity between the labeled nucleic acids may occur in the nucleic acid detection method. In other words, it has been found that a quality of a dephosphorylation reagent containing an alkaline phosphatase cannot be evaluated correctly by using the alkaline phosphatase specific activity as an index.

Thus, an object of the present invention is to provide a composition containing an alkaline phosphatase and having a high quality, a method for producing a dephosphorylated nucleic acid by using the composition and a method for producing a labeled nucleic acid by using the composition.

The present inventors have intensively studied to solve the above problem, and have found that at least any one of the following impurities can coexist in a dephosphorylation reagent containing an alkaline phosphatase (e.g., a commercially available alkaline phosphatase product):

a peptide fragment group (A) composed of two or more peptide fragments, wherein each of the two or more peptide fragments consists of 5 to 50 consecutive amino acid residues selected from positions 501 to 578 of the amino acid sequence set forth in SEQ ID NO: 5 (which corresponds to an amino acid sequence of a bovine-derived alkaline phosphatase);

a peptide fragment group (B) composed of two or more peptide fragments, wherein each of the two or more peptide fragments consists of 13 to 50 consecutive amino acid residues selected from positions 501 to 578 of the amino acid sequence set forth in SEQ ID NO: 5 and contains positions 516 to 528 of the amino acid sequence set forth in SEQ ID NO: 5;

a peptide fragment group (C) composed of two or more peptide fragments, wherein each of the two or more peptide fragments consists of 12 to 50 consecutive amino acid residues selected from positions 501 to 578 of the amino acid sequence set forth in SEQ ID NO: 5 and contains positions 534 to 545 of the amino acid sequence set forth in SEQ ID NO: 5;

a first peptide fragment consisting of the amino acid sequence set forth in SEQ ID NO: 1;

a second peptide fragment consisting of the amino acid sequence set forth in SEQ ID NO: 2;

a third peptide fragment consisting of the amino acid sequence set forth in SEQ ID NO: 3; and

a fourth peptide fragment consisting of the amino acid sequence set forth in SEQ ID NO: 4.

In addition, the present inventors have found that, by reducing, in a dephosphorylation reagent which is used to prepare a labeled nucleic acid (a labeled nucleic acid obtained by dephosphorylating the 5' end and/or the 3' end of a nucleic acid with the dephosphorylation reagent, and then binding a labeling substance to the 5' end and/or the 3' end of the dephosphorylated nucleic acid) for a nucleic acid detection method,

the content of the peptide fragment group (A),

the content of the peptide fragment group (B),

the content of the peptide fragment group (C),

the content of the second peptide fragment (preferably, the content of the second peptide fragment, and the content(s) of one, two or three peptide fragments selected from the first, third and fourth peptide fragments),

the content of the third peptide fragment (preferably, the content of the third peptide fragment, and the content(s) of one, two or three peptide fragments selected from the first, second and fourth peptide fragments), or

the content of the fourth peptide fragment (preferably, the content of the fourth peptide fragment, and the content(s) of one, two or three peptide fragments selected from the first, second and third peptide fragments),

it is possible to improve the detection sensitivity of the labeled nucleic acid in the nucleic acid detection method, thus completing the present invention. In other words, the present invention includes the following inventions.

[1] A composition containing:

an alkaline phosphatase; and

a peptide fragment group (A) composed of two or more peptide fragments, wherein each of the two or more peptide fragments consists of 5 to 50 consecutive amino acid residues selected from positions 501 to 578 of the amino acid sequence set forth in SEQ ID NO: 5,

wherein a content ratio of the peptide fragment group (A) to the alkaline phosphatase satisfies the following formula (A):

$$(X_A/Y) \times 100 \leq 4.4000 \qquad (A),$$

wherein $X_A$ represents a peak area value of the peptide fragment group (A) calculated by an automatic integration method from an extracted ion chromatogram obtained by an LC-MS/MS analysis of the composition, and Y represents a peak area value of the alkaline phosphatase calculated by an automatic integration method from a chromatogram obtained by an LC-UV analysis of the composition.

[2] The composition according to [1], wherein the peptide fragment group (A) contains one, two, three or four peptide fragments selected from the group consisting of a first peptide fragment consisting of the amino acid sequence set forth in SEQ ID NO: 1, a second peptide fragment consisting of the amino acid sequence set forth in SEQ ID NO: 2, a third peptide fragment consisting of the amino acid sequence set forth in SEQ ID NO: 3 and a fourth peptide fragment consisting of the amino acid sequence set forth in SEQ ID NO: 4.

[3] The composition according to [2], wherein:

the peptide fragment group (A) contains the first peptide fragment; and
a content ratio of the first peptide fragment to the alkaline phosphatase satisfies the following formula (1):

$$(X_1/Y) \times 100 \leq 1.0000 \qquad (1),$$

wherein $X_1$ represents a peak area value of the first peptide fragment calculated by an automatic integration method from an extracted ion chromatogram obtained by an LC-MS/MS analysis of the composition, and Y is the same as defined above.

[4] The composition according to [2] or [3], wherein:

the peptide fragment group (A) contains the second peptide fragment; and
a content ratio of the second peptide fragment to the alkaline phosphatase satisfies the following formula (2):

$$(X_2/Y) \times 100 \leq 1.6000 \qquad (2),$$

wherein $X_2$ represents a peak area value of the second peptide fragment calculated by an automatic integration method from an extracted ion chromatogram obtained by an LC-MS/MS analysis of the composition, and Y is the same as defined above.

[5] The composition according to any one of [2] to [4], wherein:

the peptide fragment group (A) contains the third peptide fragment; and
a content ratio of the third peptide fragment to the alkaline phosphatase satisfies the following formula (3):

$$(X_3/Y) \times 100 \leq 0.2000 \qquad (3),$$

wherein $X_3$ represents a peak area value of the third peptide fragment calculated by an automatic integration method from an extracted ion chromatogram obtained by an LC-MS/MS analysis of the composition, and Y is the same as defined above.

[6] The composition according to any one of [2] to [5], wherein:

the peptide fragment group (A) contains the fourth peptide fragment; and
a content ratio of the fourth peptide fragment to the alkaline phosphatase satisfies the following formula (4):

$$(X_4/Y) \times 100 \leq 0.3500 \qquad (4),$$

wherein $X_4$ represents a peak area value of the fourth peptide fragment calculated by an automatic integration method from an extracted ion chromatogram obtained by an LC-MS/MS analysis of the composition, and Y is the same as defined above.

[7] A composition containing:

an alkaline phosphatase; and
a peptide fragment group (B) composed of two or more peptide fragments, wherein each of the two or more peptide fragments consists of 13 to 50 consecutive amino acid residues selected from positions 501 to 578 of the amino acid sequence set forth in SEQ ID NO: 5 and contains positions 516 to 528 of the amino acid sequence set forth in SEQ ID NO: 5,
wherein a content ratio of the peptide fragment group (B) to the alkaline phosphatase satisfies the following formula (B):

$$(X_B/Y) \times 100 \leq 3.4000 \qquad (B),$$

wherein $X_B$ represents a peak area value of the peptide fragment group (B) calculated by an automatic integration method from an extracted ion chromatogram obtained by an LC-MS/MS analysis of the composition, and Y represents a peak area value of the alkaline phosphatase calculated by an automatic integration method from a chromatogram obtained by an LC-UV analysis of the composition.

[8] The composition according to [7], wherein the peptide fragment group (B) contains one or two peptide fragments selected from the group consisting of a first peptide fragment consisting of the amino acid sequence set forth in SEQ ID NO: 1 and a second peptide fragment consisting of the amino acid sequence set forth in SEQ ID NO: 2.

[9] The composition according to [8], wherein:

the peptide fragment group (B) contains the first peptide fragment; and
a content ratio of the first peptide fragment to the alkaline phosphatase satisfies the following formula (1):

$$(X_1/Y) \times 100 \leq 1.0000 \qquad (1),$$

wherein $X_1$ represents a peak area value of the first peptide fragment calculated by an automatic integration method from an extracted ion chromatogram obtained by an LC-MS/MS analysis of the composition, and Y is the same as defined above.

[10] The composition according to [8] or [9], wherein:

the peptide fragment group (B) contains the second peptide fragment; and
a content ratio of the second peptide fragment to the alkaline phosphatase satisfies the following formula (2):

$$(X_2/Y) \times 100 \leq 1.6000 \qquad (2),$$

wherein $X_2$ represents a peak area value of the second peptide fragment calculated by an automatic integration method from an extracted ion chromatogram obtained by an LC-MS/MS analysis of the composition, and Y is the same as defined above.

[11] A composition containing:

an alkaline phosphatase; and
a peptide fragment group (C) composed of two or more peptide fragments, wherein each of the two or more peptide fragments consists of 12 to 50 consecutive amino acid residues selected from positions 501 to 578 of the amino acid sequence set forth in SEQ ID NO: 5 and contains positions 534 to 545 of the amino acid sequence set forth in SEQ ID NO: 5,
wherein a content ratio of the peptide fragment group (C) to the alkaline phosphatase satisfies the following formula (C):

$$(X_C/Y) \times 100 \leq 1.0000 \qquad (C),$$

wherein Xc represents a peak area value of the peptide fragment group (C) calculated by an automatic integration method from an extracted ion chromatogram obtained by an LC-MS/MS analysis of the composition, and Y represents a peak area value of the alkaline phosphatase calculated by an automatic integration method from a chromatogram obtained by an LC-UV analysis of the composition.

[12] The composition according to [11], wherein the peptide fragment group (C) contains one or two peptide fragments selected from the group consisting of a third peptide fragment consisting of the amino acid sequence set forth in SEQ ID NO: 3 and a fourth peptide fragment consisting of the amino acid sequence set forth in SEQ ID NO: 4.

[13] The composition according to [12], wherein:

the peptide fragment group (C) contains the third peptide fragment; and
a content ratio of the third peptide fragment to the alkaline phosphatase satisfies the following formula (3):

$$(X_3/Y) \times 100 \leq 0.2000 \qquad (3),$$

wherein $X_3$ represents a peak area value of the third peptide fragment calculated by an automatic integration method from an extracted ion chromatogram obtained by an LC-MS/MS analysis of the composition, and Y is the same as defined above.

[14] The composition according to [12] or [13], wherein:

the peptide fragment group (C) contains the fourth peptide fragment; and
a content ratio of the fourth peptide fragment to the alkaline phosphatase satisfies the following formula (4):

$$(X_4/Y) \times 100 \leq 0.3500 \qquad (4),$$

wherein $X_4$ represents a peak area value of the fourth peptide fragment calculated by an automatic integration method from an extracted ion chromatogram obtained by an LC-MS/MS analysis of the composition, and Y is the same as defined above.

[15] A composition containing:

an alkaline phosphatase; and
a second peptide fragment consisting of the amino acid sequence set forth in SEQ ID NO: 2,
wherein a content ratio of the second peptide fragment to the alkaline phosphatase satisfies the following formula (2):

$$(X_2/Y) \times 100 \leq 1.6000 \qquad (2),$$

wherein $X_2$ represents a peak area value of the second peptide fragment calculated by an automatic integration method from an extracted ion chromatogram obtained by an LC-MS/MS analysis of the composition, and Y represents a peak area value of the alkaline phosphatase calculated by an automatic integration method from a chromatogram obtained by an LC-UV analysis of the composition.

[16] The composition according to [15], wherein:

the composition further contains a first peptide fragment consisting of the amino acid sequence set forth in SEQ ID NO: 1; and
a content ratio of the first peptide fragment to the alkaline phosphatase satisfies the following formula (1):

$$(X_1/Y) \times 100 \leq 1.0000 \qquad (1),$$

wherein $X_1$ represents a peak area value of the first peptide fragment calculated by an automatic integration method from an extracted ion chromatogram obtained by an LC-MS/MS analysis of the composition, and Y is the same as defined above.

[17] The composition according to [15] or [16], wherein:

the composition further contains a third peptide fragment consisting of the amino acid sequence set forth in SEQ ID NO: 3; and
a content ratio of the third peptide fragment to the alkaline phosphatase satisfies the following formula (3):

$$(X_3/Y) \times 100 \leq 0.2000 \qquad (3),$$

wherein $X_3$ represents a peak area value of the third peptide fragment calculated by an automatic integration method from an extracted ion chromatogram obtained by an LC-MS/MS analysis of the composition, and Y is the same as defined above.

[18] The composition according to any one of [15] to [17], wherein:

the composition further contains a fourth peptide fragment consisting of the amino acid sequence set forth in SEQ ID NO: 4; and
a content ratio of the fourth peptide fragment to the alkaline phosphatase satisfies the following formula (4):

$$(X_4/Y) \times 100 \leq 0.3500 \qquad (4),$$

wherein $X_4$ represents a peak area value of the fourth peptide fragment calculated by an automatic integration method from an extracted ion chromatogram obtained by an LC-MS/MS analysis of the composition, and Y is the same as defined above.

[19] A composition containing:

an alkaline phosphatase; and
a third peptide fragment consisting of the amino acid sequence set forth in SEQ ID NO: 3,
wherein a content ratio of the third peptide fragment to the alkaline phosphatase satisfies the following formula (3):

$$(X_3/Y) \times 100 \leq 0.2000 \qquad (3),$$

wherein $X_3$ represents a peak area value of the third peptide fragment calculated by an automatic integration method from an extracted ion chromatogram obtained by an LC-MS/MS analysis of the composition, and Y is the same as defined above.

[20] The composition according to [19], wherein:

the composition further contains a first peptide fragment consisting of the amino acid sequence set forth in SEQ ID NO: 1; and
a content ratio of the first peptide fragment to the alkaline phosphatase satisfies the following formula (1):

$$(X_1/Y) \times 100 \leq 1.0000 \qquad (1),$$

wherein $X_1$ represents a peak area value of the first peptide fragment calculated by an automatic integration method from an extracted ion chromatogram obtained by an LC-MS/MS analysis of the composition, and Y is the same as defined above.

[21] The composition according to [19] or [20], wherein:

the composition further contains a fourth peptide fragment consisting of the amino acid sequence set forth in SEQ ID NO: 4; and
a content ratio of the fourth peptide fragment to the alkaline phosphatase satisfies the following formula (4):

$$(X_4/Y) \times 100 \leq 0.3500 \qquad (4),$$

wherein $X_4$ represents a peak area value of the fourth peptide fragment calculated by an automatic integration method from an extracted ion chromatogram obtained by an LC-MS/MS analysis of the composition, and Y is the same as defined above.

[22] A composition containing:

an alkaline phosphatase; and
a fourth peptide fragment consisting of the amino acid sequence set forth in SEQ ID NO: 4,

wherein a content ratio of the fourth peptide fragment to the alkaline phosphatase satisfies the following formula (4):

$$(X_4/Y) \times 100 \leq 0.3500 \qquad (4),$$

wherein $X_4$ represents a peak area value of the fourth peptide fragment calculated by an automatic integration method from an extracted ion chromatogram obtained by an LC-MS/MS analysis of the composition, and Y is the same as defined above.

[23] The composition according to [22], wherein:

the composition further contains a first peptide fragment consisting of the amino acid sequence set forth in SEQ ID NO: 1; and
a content ratio of the first peptide fragment to the alkaline phosphatase satisfies the following formula (1):

$$(X_1/Y) \times 100 \leq 1.0000 \qquad (1),$$

wherein $X_1$ represents a peak area value of the first peptide fragment calculated by an automatic integration method from an extracted ion chromatogram obtained by an LC-MS/MS analysis of the composition, and Y is the same as defined above.

[24] The composition according to any one of [1] to [23], wherein the composition has an alkaline phosphatase specific activity of 2,000 U/mg or more.
[25] The composition according to any one of [1] to [24], wherein the alkaline phosphatase is selected from the following (a) and (b):

(a) an alkaline phosphatase containing a protein molecule consisting of the amino acid sequence set forth in SEQ ID NO: 5; and
(b) an alkaline phosphatase containing a protein molecule consisting of an amino acid sequence that has 70% or more sequence identity to the amino acid sequence set forth in SEQ ID NO: 5 and comprises positions 501 to 578 of the amino acid sequence set forth in SEQ ID NO: 5.

[26] The composition according to any one of [1] to [25], wherein the composition further contains a nucleic acid.
[27] The composition according to [26], wherein the composition is a composition used for dephosphorylating the nucleic acid.
[28] The composition according to any one of [1] to [25], wherein the composition further contains a dephosphorylated nucleic acid.
[29] The composition according to [28], wherein the composition is a composition used for preparing a labeled nucleic acid containing the dephosphorylated nucleic acid and a labeling substance bound to the dephosphorylated nucleic acid.
[30] The composition according to any one of [1] to [25], wherein the composition further contains a labeled nucleic acid containing a dephosphorylated nucleic acid and a labeling substance bound to the dephosphorylated nucleic acid.
[31] The composition according to [30], wherein the composition is a nucleic acid sample to be subjected to a nucleic acid detection method.
[32] The composition according to [31], wherein the nucleic acid detection method is a nucleic acid detection method using a nucleic acid microarray.
[33] A method for producing a dephosphorylated nucleic acid, the method including the following steps of:

providing the composition according to any one of [1] to [25];
providing a nucleic acid; and
treating the nucleic acid with the composition to dephosphorylate the nucleic acid.

[34] A method for producing a labeled nucleic acid, the method including the following steps of:

providing the composition according to any one of [1] to [25];

providing a nucleic acid;
providing a labeling substance;
treating the nucleic acid with the composition to dephosphorylate the nucleic acid; and
binding the labeling substance to the dephosphorylated nucleic acid.

The present invention provides a composition containing an alkaline phosphatase and having a high quality, a method for producing a dephosphorylated nucleic acid by using the composition and a method for producing a labeled nucleic acid by using the composition.

BRIEF DESCRIPTION OF THE DRAWINGS

FIG. 1 shows an extracted ion chromatogram on the first peptide fragment obtained by an LC-MS/MS analysis of the composition C2 in Comparative Example 2.
FIG. 2 shows an extracted ion chromatogram on the second peptide fragment obtained by an LC-MS/MS analysis of the composition C2 in Comparative Example 2.
FIG. 3 shows an extracted ion chromatogram on the third peptide fragment obtained by an LC-MS/MS analysis of the composition C2 in Comparative Example 2.
FIG. 4 shows an extracted ion chromatogram on the fourth peptide fragment obtained by an LC-MS/MS analysis of the composition C2 in Comparative Example 2.
FIG. 5 shows an extracted ion chromatogram on the first peptide fragment obtained by an LC-MS/MS analysis of the composition E1 (purified product of the composition C2) in Example 1.
FIG. 6 shows an extracted ion chromatogram on the second peptide fragment obtained by an LC-MS/MS analysis of the composition E1 (purified product of the composition C2) in Example 1.
FIG. 7 shows an extracted ion chromatogram on the third peptide fragment obtained by an LC-MS/MS analysis of the composition E1 (purified product of the composition C2) in Example 1.
FIG. 8 shows an extracted ion chromatogram on the fourth peptide fragment obtained by an LC-MS/MS analysis of the composition E1 (purified product of the composition C2) in Example 1.
FIG. 9 shows a chromatogram on an alkaline phosphatase obtained by an LC-UV analysis of the composition E1 (purified product of the composition C2) in Example 1.

DETAILED DESCRIPTION OF THE INVENTION

The present invention will be described in detail below. It is possible to combine two or more of the aspects described below, and it is possible to combine two or more of the embodiments described below. The present invention also encompasses such combinations. The expression "numerical value M to numerical value N" as used herein means a range of numerical value M or more and numerical value N or less.
The present invention provides a composition containing an alkaline phosphatase and a peptide fragment group (A) as a composition according to the first aspect.
The present invention provides a composition containing an alkaline phosphatase and a peptide fragment group (B) as a composition according to the second aspect.
The present invention provides a composition containing an alkaline phosphatase and a peptide fragment group (C) as a composition according to the third aspect.
The present invention provides a composition containing an alkaline phosphatase and a second peptide fragment consisting of the amino acid sequence set forth in SEQ ID NO: 2 as a composition according to the fourth aspect.
The present invention provides a composition containing an alkaline phosphatase and a third peptide fragment consisting of the amino acid sequence set forth in SEQ ID NO: 3 as a composition according to the fifth aspect.
The present invention provides a composition containing an alkaline phosphatase and a fourth peptide fragment consisting of the amino acid sequence set forth in SEQ ID NO: 4 as a composition according to the sixth aspect.
The compositions according to the first to sixth aspects will be collectively expressed as the "composition of the present invention." Therefore, the descriptions on the "composition of the present invention" apply to any of the compositions according to the first to sixth aspects unless otherwise specified.

[Alkaline Phosphatase]

The composition of the present invention contains an alkaline phosphatase. The composition of the present invention may contain one alkaline phosphatase or may contain two or more alkaline phosphatases.

The alkaline phosphatase contained in the composition of the present invention is not particularly limited as long as it has alkaline phosphatase activity. The alkaline phosphatase activity is activity that hydrolyzes a phosphoric monoester bond in alkalinity (pH 8 to 11, e.g., pH 8 to 10 or pH 9 to 11), and the reaction form is classified into EC3.1.3.1.

The structure of the alkaline phosphatase contained in the composition of the present invention (e.g., primary structure, secondary structure, tertiary structure, quaternary structure, etc.) is not particularly limited. For example, the alkaline phosphatase may have a sugar chain or may not have a sugar chain. The alkaline phosphatase may be any isozyme that can exist based on differences in the structure of a protein molecule (e.g., amino acid sequence of a protein molecule), glycosylation, etc. The alkaline phosphatase may be a monomer that is formed from one subunit or may be an oligomer that is formed from two or more subunits (e.g., dimer, tetramer, etc.). The oligomer may be a homooligomer or may be a heterooligomer.

The animal from which the alkaline phosphatase contained in the composition of the present invention is derived is not particularly limited. Examples of the animal from which the alkaline phosphatase is derived include a bovine, a shrimp, a microorganism into which a gene encoding an alkaline phosphatase has been introduced, and the like. Since a bovine-derived alkaline phosphatase has high alkaline phosphatase activity, the animal from which the alkaline phosphatase is derived is preferably a bovine. When the alkaline phosphatase is derived from a bovine, the organ from which the alkaline phosphatase is derived is preferably small intestine or large intestine.

The alkaline phosphatase contained in the composition of the present invention may be wild-type or may be mutated. The mutated alkaline phosphatase contains, for example, a protein molecule consisting of an amino acid sequence obtained by introducing deletion, substitution, insertion or addition of one or more amino acids to an amino acid sequence of a protein molecule of a wild-type alkaline phosphatase. The amino acid sequence of the protein molecule of the mutated alkaline phosphatase has preferably 70% or more, more preferably 75% or more, still more preferably 80% or more, yet more preferably 85% or more, further preferably 90% or more, and still further preferably 95% or more sequence identity to the amino acid sequence of the protein molecule of the wild-type alkaline phosphatase.

In a preferred embodiment, the alkaline phosphatase is selected from the following (a) and (b):

(a) an alkaline phosphatase containing a protein molecule consisting of the amino acid sequence set forth in SEQ ID NO: 5; and
(b) an alkaline phosphatase containing a protein molecule consisting of an amino acid sequence that has 70% or more sequence identity to the amino acid sequence set forth in SEQ ID NO: 5 and contains positions 501 to 578 of the amino acid sequence set forth in SEQ ID NO: 5. In this embodiment, the composition of the present invention may contain one alkaline phosphatase selected from (a) and (b), or may contain two or more alkaline phosphatases selected from (a) and (b).

The amino acid sequence of the protein molecule of the alkaline phosphatase (a) (i.e., the amino acid sequence set forth in SEQ ID NO: 5) corresponds to an amino acid sequence of a protein molecule of a bovine-derived alkaline phosphatase. Therefore, a bovine-derived alkaline phosphatase falls within the alkaline phosphatase (a).

The amino acid sequence of the protein molecule of the alkaline phosphatase (b) has preferably 70% or more, more preferably 75% or more, still more preferably 80% or more, yet more preferably 85% or more, further preferably 90% or more, and still further preferably 95% or more sequence identity to the amino acid sequence set forth in SEQ ID NO: 5.

Both of a wild-type alkaline phosphatase (e.g., an alkaline phosphatase derived from an animal other than a bovine, a bovine-derived alkaline phosphatase having a polymorphism, etc.) and a mutated alkaline phosphatase fall within the alkaline phosphatase (b). The position(s) at which one or more amino acids are deleted, substituted, inserted or added in the amino acid sequence set forth in SEQ ID NO: 5 is/are a position(s) other than positions 501 to 578 of the amino acid sequence set forth in SEQ ID NO: 5.

The alkaline phosphatases (a) and (b) can generate the peptide fragment group (A), the peptide fragment group (B), the peptide fragment group (C), the second peptide fragment, the third peptide fragment, the fourth peptide fragment, etc., by decomposition of the alkaline phosphatases.

[Peptide Fragment Group (A)]

The composition according to the first aspect contains the peptide fragment group (A). The peptide fragment group (A) is composed of two or more peptide fragments, and each peptide fragment constituting the peptide fragment group (A) consists of 5 to 50 consecutive amino acid residues selected from positions 501 to 578 of the amino acid sequence set forth in SEQ ID NO: 5. The peptide fragment group (A) is composed of, among peptide fragments contained in the composition according to the first aspect, all peptide fragments each corresponding to a peptide fragment consisting of 5 to 50 consecutive amino acid residues selected from positions 501 to 578 of the amino acid sequence set forth in SEQ ID NO: 5. In a preferred embodiment, the peptide fragment group (A) is composed of three or more peptide fragments. In a further preferred embodiment, the peptide fragment group (A) is composed of four or more peptide fragments.

The amino acid sequence of each peptide fragment constituting the peptide fragment group (A) is a part (moiety consisting of a plurality of consecutive amino acid residues) of positions 501 to 578 of the amino acid sequence set forth in SEQ ID NO: 5. In other words, the peptide fragment group (A) can be generated by decomposition of positions 501 to 578 of the amino acid sequence set forth in SEQ ID NO: 5. This does not mean that the alkaline phosphatase contained in the composition according to the first aspect is required to contain positions 501 to 578 of the amino acid sequence set forth in SEQ ID NO: 5. The alkaline phosphatase contained in the composition according to the first aspect may not contain positions 501 to 578 of the amino acid sequence set forth in SEQ ID NO: 5. However, the alkaline phosphatase contained in the composition according to the first aspect preferably contains positions 501 to 578 of the amino acid sequence set forth in SEQ ID NO: 5.

The amino acid sequence of each peptide fragment constituting the peptide fragment group (A) is not particularly limited as long as the amino acid sequence is a part of positions 501 to 578 of the amino acid sequence set forth in SEQ ID NO: 5. The amino acid sequence of each peptide fragment constituting the peptide fragment group (A) is preferably a part of positions 505 to 578 of the amino acid sequence set forth in SEQ ID NO: 5, more preferably a part of positions 511 to 578 of the amino acid sequence set forth in SEQ ID NO: 5, and still more preferably a part of positions 511 to 571 of the amino acid sequence set forth in SEQ ID NO: 5.

The number of amino acid residues constituting each peptide fragment constituting the peptide fragment group (A) is not particularly limited as long as the number is 5 to 50, and the number is preferably 5 to 45, more preferably 10 to 40, and still more preferably 10 to 30.

In a preferred embodiment, the peptide fragment group (A) contains one, two, three or four peptide fragments selected from the group consisting of the first peptide fragment consisting of the amino acid sequence set forth in SEQ ID NO: 1, the second peptide fragment consisting of the amino acid sequence set forth in SEQ ID NO: 2, the third peptide fragment consisting of the amino acid sequence set forth in SEQ ID NO: 3 and the fourth peptide fragment consisting of the amino acid sequence set forth in SEQ ID NO: 4. In this embodiment, the peptide fragment group (A) may or may not contain a peptide fragment other than the first to fourth peptide fragments.

The amino acid sequence set forth in SEQ ID NO: 1 (VPLASETHGGEDVAVF) corresponds to positions 516 to 531 of the amino acid sequence set forth in SEQ ID NO: 5. The amino acid sequence set forth in SEQ ID NO: 2 (VPLASETHGGEDV) corresponds to positions 516 to 528 of the amino acid sequence set forth in SEQ ID NO: 5. The amino acid sequence set forth in SEQ ID NO: 3 (GPQAHLVHGVQEETFVAH) corresponds to positions 534 to 551 of the amino acid sequence set forth in SEQ ID NO: 5. The amino acid sequence set forth in SEQ ID NO: 4 (GPQAHLVHGVQE) corresponds to positions 534 to 545 of the amino acid sequence set forth in SEQ ID NO: 5.

The peptide fragment group (A) can be generated by decomposition of an alkaline phosphatase containing positions 501 to 578 of the amino acid sequence set forth in SEQ ID NO: 5. The peptide fragment group (A) may be one generated by decomposition of an alkaline phosphatase not contained in the composition according to the first aspect, but is usually one generated by decomposition of an alkaline phosphatase contained in the composition according to the first aspect. Therefore, in a preferred embodiment, the alkaline phosphatase contained in the composition according to the first aspect is an alkaline phosphatase that can generate the peptide fragment group (A). In a preferred embodiment, the alkaline phosphatase that can generate the peptide fragment group (A) is selected from the alkaline phosphatases (a) and (b). In this embodiment, the composition according to the first aspect contains one or two or more alkaline phosphatases selected from the alkaline phosphatases (a) and (b).

[Peptide Fragment Group (B)]

The composition according to the second aspect contains the peptide fragment group (B). The peptide fragment group (B) is composed of two or more peptide fragments, and each peptide fragment constituting the peptide fragment group (B) consists of 13 to 50 consecutive amino acid residues selected from positions 501 to 578 of the amino acid sequence set forth in SEQ ID NO: 5 and contains positions 516 to 528 (VPLASETHGGEDV) of the amino acid sequence set forth in SEQ ID NO: 5. The peptide fragment group (B) is composed of, among peptide fragments contained in the composition according to the second aspect, all peptide fragments each corresponding to a peptide fragment consisting of 13 to 50 consecutive amino acid residues selected from positions 501 to 578 of the amino acid sequence set forth in SEQ ID NO: 5 and containing positions 516 to 528 of the amino acid sequence set forth in SEQ ID NO: 5.

In the amino acid sequence of each peptide fragment constituting the peptide fragment group (B), the position at which positions 516 to 528 of the amino acid sequence set forth in SEQ ID NO: 5 are contained is not particularly limited. The position at which positions 516 to 528 of the amino acid sequence set forth in SEQ ID NO: 5 are contained may be any of the N terminal part of a peptide fragment, the C terminal part of a peptide fragment, and a part other than the N terminal part and the C terminal part of a peptide fragment.

The amino acid sequence of each peptide fragment constituting the peptide fragment group (B) is a part (moiety consisting of a plurality of consecutive amino acid residues) of positions 501 to 578 of the amino acid sequence set forth in SEQ ID NO: 5. In other words, the peptide fragment group (B) can be generated by decomposition of positions 501 to 578 of the amino acid sequence set forth in SEQ ID NO: 5. This does not mean that the alkaline phosphatase contained in the composition according to the second aspect is required to contain positions 501 to 578 of the amino acid sequence set forth in SEQ ID NO: 5. The alkaline phosphatase contained in the composition according to the second aspect may not contain positions 501 to 578 of the amino acid sequence set forth in SEQ ID NO: 5. However, the alkaline phosphatase contained in the composition according to the second aspect preferably contains positions 501 to 578 of the amino acid sequence set forth in SEQ ID NO: 5.

The amino acid sequence of each peptide fragment constituting the peptide fragment group (B) is not particularly limited as long as the amino acid sequence is a part of positions 501 to 578 of the amino acid sequence set forth in SEQ ID NO: 5 and contains positions 516 to 528 of the amino acid sequence set forth in SEQ ID NO: 5. The amino acid sequence of each peptide fragment constituting the peptide fragment group (B) is preferably a part of positions 501 to 570 of the amino acid sequence set forth in SEQ ID NO: 5, more preferably a part of positions 501 to 560 of the amino acid sequence set forth in SEQ ID NO: 5, and still more preferably a part of positions 501 to 555 of the amino acid sequence set forth in SEQ ID NO: 5.

The number of amino acid residues constituting each peptide fragment constituting the peptide fragment group (B) is not particularly limited as long as the number is 13 to 50, and the number is preferably 13 to 45, more preferably 13 to 40, and still more preferably 13 to 35.

In a preferred embodiment, the peptide fragment group (B) contains one or two peptide fragments selected from the group consisting of the first peptide fragment consisting of the amino acid sequence set forth in SEQ ID NO: 1 and the second peptide fragment consisting of the amino acid sequence set forth in SEQ ID NO: 2. In this embodiment, the peptide fragment group (B) may or may not contain a peptide fragment other than the first and second peptide fragments.

The amino acid sequence set forth in SEQ ID NO: 1 (<u>VPLASETHGGED</u>VAVF) corresponds to positions 516 to 531 of the amino acid sequence set forth in SEQ ID NO: 5 and contains positions 516 to 528 of the amino acid sequence set forth in SEQ ID NO: 5 (the underlined part corresponds to positions 516 to 528 of the amino acid sequence set forth in SEQ ID NO: 5). The amino acid sequence set forth in SEQ ID NO: 2 (VPLASETHGGEDV) corresponds to positions 516 to 528 of the amino acid sequence set forth in SEQ ID NO: 5.

The peptide fragment group (B) can be generated by decomposition of an alkaline phosphatase containing positions 501 to 578 of the amino acid sequence set forth in SEQ ID NO: 5. The peptide fragment group (B) may be one generated by decomposition of an alkaline phosphatase not contained in the composition according to the second aspect, but is usually one generated by decomposition of an alkaline phosphatase contained in the composition according to the second aspect. Therefore, in a preferred embodiment, the alkaline phosphatase contained in the composition according to the second aspect is an alkaline phosphatase that can generate the peptide fragment group (B). In a preferred embodiment, the alkaline phosphatase that can generate the peptide fragment group (B) is selected from the alkaline phosphatases (a) and (b). In this embodiment, the composition according to the second aspect contains one or two or more alkaline phosphatases selected from the alkaline phosphatases (a) and (b).

[Peptide Fragment Group (C)]

The composition according to the third aspect contains a peptide fragment group (C). The peptide fragment group (C) is composed of two or more peptide fragments, and each peptide fragment constituting the peptide fragment group (C) consists of 12 to 50 consecutive amino acid residues selected from positions 501 to 578 of the amino acid sequence set forth in SEQ ID NO: 5 and contains positions 534 to 545 (GPQAHLVHGVQE) of the amino acid sequence set forth in SEQ ID NO: 5. The peptide fragment group (C) is composed of, among peptide fragments contained in the composition according to the third aspect, all peptide fragments each corresponding to a peptide fragment consisting of 12 to 50 consecutive amino acid residues selected from positions 501 to 578 of the amino acid sequence set forth in SEQ ID NO: 5 and containing positions 534 to 545 of the amino acid sequence set forth in SEQ ID NO: 5.

In the amino acid sequence of each peptide fragment constituting the peptide fragment group (C), the position at which positions 534 to 545 of the amino acid sequence set forth in SEQ ID NO: 5 may be any of the N terminal part of a peptide fragment, the C terminal part of a peptide fragment, and a part other than the N terminal part and the C terminal part of a peptide fragment.

The amino acid sequence of each peptide fragment constituting the peptide fragment group (C) is a part (moiety consisting of a plurality of consecutive amino acid residues) of positions 501 to 578 of the amino acid sequence set forth in SEQ ID NO: 5. In other words, the peptide fragment group (C) can be generated by decomposition of positions 501 to 578 of the amino acid sequence set forth in SEQ ID NO: 5. This does not mean that the alkaline phosphatase contained in the composition according to the third aspect is required to contain positions 501 to 578 of the amino acid sequence set forth in SEQ ID NO: 5. The alkaline phosphatase contained in the composition according to the third aspect may not contain positions 501 to 578 of the amino acid sequence set forth in SEQ ID NO: 5. However, the alkaline phosphatase contained in the composition according to the third aspect preferably contains positions 501 to 578 of the amino acid sequence set forth in SEQ ID NO: 5.

The amino acid sequence of each peptide fragment constituting the peptide fragment group (C) is not particularly limited as long as the amino acid sequence is a part of positions 501 to 578 of the amino acid sequence set forth in SEQ ID NO: 5 and contains positions 534 to 545 of the amino acid sequence set forth in SEQ ID NO: 5. The amino acid sequence of each peptide fragment constituting the peptide fragment group (C) is preferably a part of positions 506 to 578 of the amino acid sequence set forth in SEQ ID NO: 5, more preferably a part of positions 511 to 578 of the amino acid sequence set forth in SEQ ID NO: 5, and still more preferably a part of positions 521 to 578 of the amino acid sequence set forth in SEQ ID NO: 5.

The number of amino acid residues constituting each peptide fragment constituting the peptide fragment group (C) is not particularly limited as long as the number is 12 to 50, and the number is preferably 12 to 45, more preferably 12 to 40, and still more preferably 12 to 35.

In a preferred embodiment, the peptide fragment group (C) contains one or two peptide fragments selected from the group consisting of the third peptide fragment consisting of the amino acid sequence set forth in SEQ ID NO: 3 and the fourth peptide fragment consisting of the amino acid sequence set forth in SEQ ID NO: 4. In this embodiment, the peptide fragment group (C) may or may not contain a peptide fragment other than the third and fourth peptide fragments.

The amino acid sequence set forth in SEQ ID NO: 3 (GPQAHLVHGVQEETFVAH) corresponds to positions 534 to 551 of the amino acid sequence set forth in SEQ ID NO: 5 and contains positions 534 to 545 of the amino acid sequence set forth in SEQ ID NO: 5 (the underlined part corresponds to positions 534 to 545 of the amino acid sequence set forth in SEQ ID NO: 5). The amino acid sequence set forth in SEQ ID NO: 4 (GPQAHLVHGVQE) corresponds to positions 534 to 545 of the amino acid sequence set forth in SEQ ID NO: 5.

The peptide fragment group (C) can be generated by decomposition of an alkaline phosphatase containing positions 501 to 578 of the amino acid sequence set forth in SEQ ID NO: 5. The peptide fragment group (C) may be one generated by decomposition of an alkaline phosphatase not contained in the composition according to the third aspect, but is usually one generated by decomposition of an alkaline phosphatase contained in the composition according to the third aspect. Therefore, in a preferred embodiment, the alkaline phosphatase contained in the composition according to the third aspect is an alkaline phosphatase that can generate the peptide fragment group (C). In a preferred embodiment, the alkaline phosphatase that can generate the peptide fragment group (C) is selected from the alkaline phosphatases (a) and (b). In this embodiment, the composition according to the third aspect contains one or two or more alkaline phosphatases selected from the alkaline phosphatases (a) and (b).

[First Peptide Fragment]

In a preferred embodiment, the composition according to the first, second, third, fourth, fifth or sixth aspect contains the first peptide fragment consisting of the amino acid sequence set forth in SEQ ID NO: 1.

The first peptide fragment can be generated by decomposition of an alkaline phosphatase containing positions 501 to 578 of the amino acid sequence set forth in SEQ ID NO: 5. The first peptide fragment may be one generated by decomposition of an alkaline phosphatase not contained in the composition according to the first, second, third, fourth, fifth or sixth aspect, but is usually one generated by decomposition of an alkaline phosphatase contained in the composition according to the first, second, third, fourth, fifth or sixth aspect. Therefore, in a preferred embodiment, the alkaline phosphatase contained in the composition according to the first, second, third, fourth, fifth or sixth aspect is an alkaline phosphatase that can generate the first peptide fragment. In a preferred embodiment, the alkaline phosphatase that can generate the first peptide fragment is selected from the alkaline phosphatases (a) and (b). In this embodiment, the composition according to the first, second, third, fourth, fifth or sixth aspect contains one or two or more alkaline phosphatases selected from the alkaline phosphatases (a) and (b).

[Second Peptide Fragment]

The composition according to the fourth aspect contains the second peptide fragment consisting of the amino acid sequence set forth in SEQ ID NO: 2. In a preferred embodiment, the composition according to the fourth aspect further contains one, two or three peptide fragments selected from the group consisting of the first peptide fragment consisting of the amino acid sequence set forth in SEQ ID NO: 1, the third peptide fragment consisting of the amino acid sequence set forth in SEQ ID NO: 3 and the fourth peptide fragment consisting of the amino acid sequence set forth in SEQ ID NO: 4. In this embodiment, the composition according to the fourth aspect may or may not contain a peptide fragment other than the first to fourth peptide fragments.

The second peptide fragment can be generated by decomposition of an alkaline phosphatase containing positions 501 to 578 of the amino acid sequence set forth in SEQ ID NO: 5. The second peptide fragment may be one generated by decomposition of an alkaline phosphatase not contained in the composition according to the fourth aspect, but is usually one generated by decomposition of an alkaline phosphatase contained in the composition according to the fourth aspect. Therefore, in a preferred embodiment, the alkaline phosphatase contained in the composition according to the fourth aspect is an alkaline phosphatase that can generate the second peptide fragment. In a preferred embodiment, the alkaline phosphatase that can generate the second peptide fragment is selected from the alkaline phosphatases (a) and (b). In this embodiment, the composition according to the fourth aspect contains one or two or more alkaline phosphatases selected from the alkaline phosphatases (a) and (b).

[Third Peptide Fragment]

The composition according to the fifth aspect contains the third peptide fragment consisting of the amino acid sequence set forth in SEQ ID NO: 3. In a preferred embodiment, the composition according to the fifth aspect further contains one, two or three peptide fragments selected from the group consisting of the first peptide fragment consisting of the amino acid sequence set forth in SEQ ID NO: 1, the second peptide fragment consisting of the amino acid sequence set forth in SEQ ID NO: 2 and the fourth peptide fragment consisting of the amino acid sequence set forth in SEQ ID NO: 4. In this embodiment, the composition according to the fifth aspect may or may not contain a peptide fragment other than the first to fourth peptide fragments.

The third peptide fragment can be generated by decomposition of an alkaline phosphatase containing positions 501 to 578 of the amino acid sequence set forth in SEQ ID NO: 5. The third peptide fragment may be one generated by decomposition of an alkaline phosphatase not contained in the composition according to the fifth aspect, but is usually one generated by decomposition of an alkaline phosphatase contained in the composition according to the fifth aspect. Therefore, in a preferred embodiment, the alkaline phosphatase contained in the composition according to the fifth aspect is an alkaline phosphatase that can generate the third peptide fragment. In a preferred embodiment, the alkaline phosphatase that can generate the third peptide fragment is selected from the alkaline phosphatases (a) and (b). In this embodiment, the composition according to the fifth aspect contains one or two or more alkaline phosphatases selected from the alkaline phosphatases (a) and (b).

[Fourth Peptide Fragment]

The composition according to the sixth aspect contains the fourth peptide fragment consisting of the amino acid sequence set forth in SEQ ID NO: 4. In a preferred embodiment, the composition according to the sixth aspect further contains one, two or three peptide fragments selected from the group consisting of the first peptide fragment consisting of the amino acid sequence set forth in SEQ ID NO: 1, the second peptide fragment consisting of the amino acid sequence set forth in SEQ ID NO: 2 and the third peptide fragment consisting of the amino acid sequence set forth in SEQ ID NO: 3. In this embodiment, the composition according to the sixth aspect may or may not contain a peptide fragment other than the first to fourth peptide fragments.

The fourth peptide fragment can be generated by decomposition of an alkaline phosphatase containing positions 501 to 578 of the amino acid sequence set forth in SEQ ID NO: 5. The fourth peptide fragment may be one generated by decomposition of an alkaline phosphatase not contained in the composition according to the sixth aspect, but is usually one generated by decomposition of an alkaline phosphatase contained in the composition according to the sixth aspect. Therefore, in a preferred embodiment, the alkaline phosphatase contained in the composition according to the sixth aspect is an alkaline phosphatase that can generate the fourth peptide fragment. In a preferred embodiment, the alkaline phosphatase that can generate the fourth peptide fragment is selected from the alkaline phosphatases (a) and (b). In this embodiment, the composition according to the sixth aspect contains one or two or more alkaline phosphatases selected from the alkaline phosphatases (a) and (b).

[Content Ratio of Peptide Fragment Group (A) to Alkaline Phosphatase]

In the composition according to the first aspect, the content ratio of the peptide fragment group (A) to the alkaline phosphatase satisfies the following formula (A):

$$(X_A/Y) \times 100 \leq 4.4000 \qquad (A).$$

In the formula (A), $X_A$ represents a peak area value of the peptide fragment group (A) calculated by an automatic integration method from an extracted ion chromatogram obtained by an LC-MS/MS analysis of the composition according to the first aspect, and Y represents a peak area value of the alkaline phosphatase calculated by an automatic integration

method from a chromatogram obtained by an LC-UV analysis of the composition according to the first aspect. The "peak area value of the peptide fragment group (A)" means a total peak area value of all peptide fragments constituting the peptide fragment group (A). The "peak area value of the alkaline phosphatase" means, when the composition according to the first aspect contains one alkaline phosphatase, a peak area value of the one alkaline phosphatase, and, when the composition according to the first aspect contains two or more alkaline phosphatases, a total peak area value of the two or more alkaline phosphatases (i.e., total peak area value of all alkaline phosphatases contained in the composition according to the first aspect).

The LC-MS/MS analysis and the LC-UV analysis are performed by using a sample in which the content ratio of the peptide fragment group (A) to the alkaline phosphatase is the same as that of the composition according to the first aspect. The LC-MS/MS analysis and the LC-UV analysis can be performed, for example, by using an aqueous solution prepared from the composition according to the first aspect and with an alkaline phosphatase concentration of 10% by weight.

The LC-MS/MS analysis is one of the hyphenated methods. The hyphenated method is a method for analyzing by connecting a chromatograph such as a gas chromatograph and a liquid chromatograph to a mass spectrometer. The LC-MS/MS analysis is a method for analyzing by connecting a liquid chromatograph (LC) to a tandem mass spectrometer (MS/MS). In the LC-MS/MS analysis, analyte components separated by the liquid chromatograph are ionized, the ions thus produced are separated by the tandem mass spectrometer, and specific mass ions are fragmented and detected.

In the hyphenated method, an extracted ion chromatogram is a chromatogram expressed as a function of time obtained by measuring a mass spectrum at a certain time interval and storing it in a computer, followed by reading a relative intensity at a specific (not necessarily one type) m/z value. The m/z value of an ion of each peptide fragment constituting the peptide fragment group (A) used for detecting a peak of each peptide fragment constituting the peptide fragment group (A) is preferably 50 to 2,200, more preferably 200 to 1,500, and still more preferably 300 to 1,200. Regarding an ion of a peptide fragment with an m/z value being specified, it is possible to create an extracted ion chromatogram of the ion of the peptide fragment based on the m/z value. The m/z value of the first peptide fragment is 814.4018, the m/z value of the second peptide fragment is 655.8148, the m/z value of the third peptide fragment is 652.6623, and the m/z value of the fourth peptide fragment is 636.3282. Regarding a peptide fragment with an m/z value not being specified, after whether a certain peak corresponds to a peak of a predetermined peptide fragment or not is confirmed by an amino acid sequence analysis of a peptide fragment showing the peak, it is possible to create an extracted ion chromatogram of the peptide fragment based on the m/z value of the peak.

The LC-UV analysis is a method for analyzing by connecting a liquid chromatograph (LC) to an ultraviolet detector (UV detector). In the LC-UV analysis, an alkaline phosphatase is detected as a component having absorption at 214 nm.

Conditions of the LC-MS/MS analysis are as follows.

<Conditions of LC-MS/MS Analysis>

<Apparatus Configuration>

Mass spectrometer: maXis impact (manufactured by Bruker Daltnics, Inc.)

<Conditions of Mass Spectrometry>

Ionization method: ESI
Measured ion: cation
Capillary voltage: 4,500 V
Nebulizer: 2.0 bar
Dry gas: 8.0 L/min
Detector voltage: 1,823 V
Measuring span (MS): m/z 50 to 2,200

<MS/MS Conditions>

Measuring span (MS): m/z 50 to 2,200
Collision gas: nitrogen

<Conditions of LC-UV Analysis>

<Apparatus Configuration>

Liquid chromatograph: LC-30A system (manufactured by Shimadzu Corporation)
Detector: UV-Vis (190 to 900 nm, manufactured by Shimadzu Corporation)

<Conditions of Liquid Chromatography>

Column: Acquity BEH C18 1.7 $\mu$m (manufactured by Waters Corporation)

Column size: 2.1 mm $\times$ 100 mm
Column temperature: 50°C
Mobile phase flow rate: 0.2 mL/min
Mobile phase A: mixed solution of water/formic acid (1000:1)
Mobile phase B: mixed solution of acetonitrile/water/formic acid (900:100:1)
Injection volume: 20 $\mu$L
Gradient program:

[Table 1]

| Times (min) | Mobile phase A (vol%) | Mobile phase B (vol%) |
|---|---|---|
| 0 | 100 | 0 |
| 10 | 100 | 0 |
| 40 | 35 | 65 |
| 40.1 | 0 | 100 |
| 50 | 0 | 100 |
| 50.1 | 100 | 0 |
| 60 | 100 | 0 |

The value of $(X_A/Y) \times 100$ is not particularly limited as long as it is 4.4000 or less, and the smaller the value is, the more preferable it is. The value of $(X_A/Y) \times 100$ is preferably 4.0000 or less, more preferably 3.5000 or less, and still more preferably 3.0000 or less. The lower limit of $(X_A/Y) \times 100$ is a detection limit. In terms of obtaining an effect that matches an effort to decrease the value of $(X_A/Y) \times 100$ (e.g., removal and separation of the peptide fragment group (A) by purification), the value of $(X_A/Y) \times 100$ is preferably 0.0800 or more, more preferably 0.1000 or more, and still more preferably 0.1500 or more.
The smaller the peak area value of the peptide fragment group (A), which is calculated by an automatic integration method from an extracted ion chromatogram obtained by an LC-MS/MS analysis performed by using an aqueous solution prepared from the composition according to the first aspect and with an alkaline phosphatase concentration of 10% by weight, is, the more preferable it is. The peak area value of the peptide fragment group (A) is preferably 10,000 or less, more preferably 8,000 or less, and still more preferably 7,000 or less. The lower limit of the peak area value of the peptide fragment group (A) is a detection limit. In terms of obtaining an effect that matches an effort to decrease the peak area value of the peptide fragment group (A) (e.g., removal and separation of the peptide fragment group (A) by purification), the peak area value of the peptide fragment group (A) is preferably 600 or more, more preferably 800 or more, and still more preferably 1,000 or more.
The peak area value of an alkaline phosphatase, which is calculated by an automatic integration method from a chromatogram obtained by an LC-UV analysis performed by using an aqueous solution prepared from the composition according to the first aspect and with an alkaline phosphatase concentration of 10% by weight, is preferably 200,000 or more, more preferably 220,000 or more, and still more preferably 240,000 or more. The upper limit of the peak area value of an alkaline phosphatase is not particularly limited. The peak area value of an alkaline phosphatase is preferably 500,000 or less, more preferably 400,000 or less, and still more preferably 350,000 or less.

[Content Ratio of Peptide Fragment Group (B) to Alkaline Phosphatase]

In the composition according to the second aspect, the content ratio of the peptide fragment group (B) to the alkaline phosphatase satisfies the following formula (B):

$$(X_B/Y) \times 100 \leq 3.4000 \qquad (B).$$

In the formula (B), $X_B$ represents a peak area value of the peptide fragment group (B) calculated by an automatic integration method from an extracted ion chromatogram obtained by an LC-MS/MS analysis of the composition according to the second aspect, and Y represents a peak area value of the alkaline phosphatase calculated by an automatic integration method from a chromatogram obtained by an LC-UV analysis of the composition according to the second aspect. The "peak area value of the peptide fragment group (B)" means a total peak area value of all peptide fragments constituting the peptide fragment group (B). The "peak area value of the alkaline phosphatase" means, when the composition according to the second aspect contains one alkaline phosphatase, a peak area value of the one alkaline phosphatase, and, when the composition according to the second aspect contains two or more alkaline phosphatases, a total peak area value of the two or more alkaline phosphatases (i.e., total peak area value of all alkaline phosphatases contained in the composition according to the second aspect). The descriptions on the formula (A) (including the descriptions on the LC-MS/MS analysis and the LC-UV analysis) also apply to the formula (B) unless otherwise specified.

The m/z value of an ion of each peptide fragment constituting the peptide fragment group (B) used for detecting a peak of each peptide fragment constituting the peptide fragment group (B) is preferably 50 to 2,200, more preferably 200 to 1,500, and still more preferably 300 to 1,200.

The value of $(X_B/Y) \times 100$ is not particularly limited as long as it is 3.4000 or less, and the smaller the value is, the more preferable it is. The value of $(X_B/Y) \times 100$ is preferably 3.0000 or less, more preferably 2.8000 or less, and still more preferably 2.5000 or less. The lower limit of $(X_B/Y) \times 100$ is a detection limit. In terms of obtaining an effect that matches an effort to decrease the value of $(X_B/Y) \times 100$ (e.g., removal and separation of the peptide fragment group (B) by purification), the value of $(X_B/Y) \times 100$ is preferably 0.0800 or more, more preferably 0.1000 or more, and still more preferably 0.1500 or more.

The smaller the peak area value of the peptide fragment group (B), which is calculated by an automatic integration method from an extracted ion chromatogram obtained by an LC-MS/MS analysis performed by using an aqueous solution prepared from the composition according to the second aspect and with an alkaline phosphatase concentration of 10% by weight, is, the more preferable it is. The peak area value of the peptide fragment group (B) is preferably 7,500 or less, more preferably 7,000 or less, and still more preferably 6,000 or less. The lower limit of the peak area value of the peptide fragment group (B) is a detection limit. In terms of obtaining an effect that matches an effort to decrease the peak area value of the peptide fragment group (B) (e.g., removal and separation of the peptide fragment group (B) by purification), the peak area value of the peptide fragment group (B) is preferably 500 or more, more preferably 600 or more, and still more preferably 800 or more.

The peak area value of an alkaline phosphatase, which is calculated by an automatic integration method from a chromatogram obtained by an LC-UV analysis performed by using an aqueous solution prepared from the composition according to the second aspect and with an alkaline phosphatase concentration of 10% by weight, is preferably 200,000 or more, more preferably 220,000 or more, and still more preferably 240,000 or more. The upper limit of the peak area value of an alkaline phosphatase is not particularly limited. The peak area value of an alkaline phosphatase is preferably 500,000 or less, more preferably 400,000 or less, and still more preferably 350,000 or less.

In an embodiment in which the composition according to the second aspect contains a peptide fragment consisting of 5 to 50 consecutive amino acid residues selected from positions 501 to 578 of the amino acid sequence set forth in SEQ ID NO: 5 other than the peptide fragment group (B), the composition according to the second aspect may or may not satisfy the formula (A).

[Content Ratio of Peptide Fragment Group (C) to Alkaline Phosphatase]

In the composition according to the third aspect, the content ratio of the peptide fragment group (C) to the alkaline phosphatase satisfies the following formula (C):

$$(X_C/Y) \times 100 \leq 1.0000 \qquad (C).$$

In the formula (C), Xc represents a peak area value of the peptide fragment group (C) calculated by an automatic integration method from an extracted ion chromatogram obtained by an LC-MS/MS analysis of the composition according to the third aspect, and Y represents a peak area value of the alkaline phosphatase calculated by an automatic integration method from a chromatogram obtained by an LC-UV analysis of the composition according to the third aspect. The "peak area value of the peptide fragment group (C)" means a total peak area value of all peptide fragments constituting the peptide fragment group (C). The "peak area value of the alkaline phosphatase" means, when the composition according to the third aspect contains one alkaline phosphatase, a peak area value of the one alkaline phosphatase, and, when the composition according to the third aspect contains two or more alkaline phosphatases, a total peak area value of the two or more alkaline phosphatases (i.e., total peak area value of all alkaline phosphatases contained in the composition according to the third aspect). The descriptions on the formula (A) (including the descriptions on the LC-MS/MS analysis and the LC-UV analysis) also apply to the formula (C) unless otherwise specified.

The m/z value of an ion of each peptide fragment constituting the peptide fragment group (C) used for detecting a peak of each peptide fragment constituting the peptide fragment group (C) is preferably 50 to 2,200, more preferably 200 to 1,500, and still more preferably 300 to 1,200.

The value of $(X_C/Y) \times 100$ is not particularly limited as long as it is 1.0000 or less, and the smaller the value is, the more preferable it is. The value of $(X_C/Y) \times 100$ is preferably 0.9000 or less, more preferably 0.8000 or less, and still more preferably 0.7000 or less. The lower limit of $(X_C/Y) \times 100$ is a detection limit. In terms of obtaining an effect that matches an effort to decrease the value of $(X_C/Y) \times 100$ (e.g., removal and separation of the peptide fragment group (C) by purification), the value of $(X_C/Y) \times 100$ is preferably 0.0800 or more, more preferably 0.1000 or more, and still more preferably 0.1500 or more.

The smaller the peak area value of the peptide fragment group (C), which is calculated by an automatic integration method from an extracted ion chromatogram obtained by an LC-MS/MS analysis performed by using an aqueous solution prepared from the composition according to the third aspect and with an alkaline phosphatase concentration of 10% by weight, is, the more preferable it is. The peak area value of the peptide fragment group (C) is preferably 2,400 or less, more preferably 2,000 or less, and still more preferably 1,500 or less. The lower limit of the peak area value of the peptide fragment group (C) is a detection limit. In terms of obtaining an effect that matches an effort to decrease the peak area value of the peptide fragment group (C) (e.g., removal and separation of the peptide fragment group (C) by purification), the peak area value of the peptide fragment group (C) is preferably 200 or more, more preferably 300 or more, and still more preferably 400 or more.

The peak area value of an alkaline phosphatase, which is calculated by an automatic integration method from a chromatogram obtained by an LC-UV analysis performed by using an aqueous solution prepared from the composition according to the third aspect and with an alkaline phosphatase concentration of 10% by weight, is preferably 200,000 or more, more preferably 220,000 or more, and still more preferably 240,000 or more. The upper limit of the peak area value of an alkaline phosphatase is not particularly limited. The peak area value of an alkaline phosphatase is preferably 500,000 or less, more preferably 400,000 or less, and still more preferably 350,000 or less.

In an embodiment in which the composition according to the third aspect contains a peptide fragment consisting of 5 to 50 consecutive amino acid residues selected from positions 501 to 578 of the amino acid sequence set forth in SEQ ID NO: 5 other than the peptide fragment group (C), the composition according to the third aspect may or may not satisfy the formula (A).

[Content Ratio of First Peptide Fragment to Alkaline Phosphatase]

In an embodiment in which the composition according to the first, second, third, fourth, fifth or sixth aspect contains the first peptide fragment, the content ratio of the first peptide fragment to the alkaline phosphatase preferably satisfies the following formula (1):

$$(X_1/Y) \times 100 \leq 1.0000 \qquad (1).$$

In the formula (1), $X_1$ represents a peak area value of the first peptide fragment calculated by an automatic integration method from an extracted ion chromatogram obtained by an LC-MS/MS analysis of the composition according to the first, second, third, fourth, fifth or sixth aspect, and Y represents a peak area value of the alkaline phosphatase calculated by an automatic integration method from a chromatogram obtained by an LC-UV analysis of the composition according to the first, second, third, fourth, fifth or sixth aspect. The descriptions on the formula (A) (including the descriptions on the LC-MS/MS analysis and the LC-UV analysis) also apply to the formula (1) unless otherwise specified.

The value of $(X_1/Y) \times 100$ is not particularly limited as long as it is 1.0000 or less, and the smaller the value is, the more preferable it is. The value of $(X_1/Y) \times 100$ is preferably 0.9000 or less, more preferably 0.8000 or less, and still more preferably 0.7000 or less. The lower limit of $(X_1/Y) \times 100$ is a detection limit. In terms of obtaining an effect that matches an effort to decrease the value of $(X_1/Y) \times 100$ (e.g., removal and separation of the first peptide fragment by purification), the value of $(X_1/Y) \times 100$ is preferably 0.0500 or more, more preferably 0.0600 or more, and still more preferably 0.0700 or more.

The smaller the peak area value of the first peptide fragment calculated by an automatic integration method from an extracted ion chromatogram obtained by an LC-MS/MS analysis performed by using an aqueous solution prepared from the composition according to the first, second, third, fourth, fifth or sixth aspect and with an alkaline phosphatase concentration of 10% by weight is, the more preferable it is. The peak area value of the first peptide fragment is preferably 3,000 or less, more preferably 2,500 or less, and still more preferably 2,000 or less. The lower limit of the peak area value of the first peptide fragment is a detection limit. In terms of obtaining an effect that matches an effort to decrease the peak area value of the first peptide fragment (e.g., removal and separation of the first peptide fragment by purification), the peak area value of the first peptide fragment is preferably 100 or more, more preferably 150 or more, and still more preferably 200 or more.

The peak area value of an alkaline phosphatase calculated by an automatic integration method from a chromatogram obtained by an LC-UV analysis performed by using an aqueous solution prepared from the composition according to the first, second, third, fourth, fifth or sixth aspect and with an alkaline phosphatase concentration of 10% by weight is preferably 200,000 or more, more preferably 220,000 or more, and still more preferably 240,000 or more. The upper limit of the peak area value of an alkaline phosphatase is not particularly limited. The peak area value of an alkaline phosphatase is preferably 500,000 or less, more preferably 400,000 or less, and still more preferably 350,000 or less.

[Content Ratio of Second Peptide Fragment to Alkaline Phosphatase]

In the composition according to the fourth aspect, the content ratio of the second peptide fragment to the alkaline phosphatase satisfies the following formula (2):

$$(X_2/Y) \times 100 \leq 1.6000 \qquad (2).$$

In an embodiment in which the composition according to the first, second, third, fifth or sixth aspect contains the second peptide fragment, the content ratio of the second peptide fragment to the alkaline phosphatase preferably satisfies the following formula (2):

$$(X_2/Y) \times 100 \leq 1.6000 \qquad (2).$$

In the formula (2), $X_2$ represents a peak area value of the second peptide fragment calculated by an automatic integration method from an extracted ion chromatogram obtained by an LC-MS/MS analysis of the composition according to the first, second, third, fourth, fifth or sixth aspect, and Y represents a peak area value of the alkaline phosphatase calculated by an automatic integration method from a chromatogram obtained by an LC-UV analysis of the composition according to the first, second, third, fourth, fifth or sixth aspect. The descriptions on the formula (A) (including the descriptions on the LC-MS/MS analysis and the LC-UV analysis) also apply to the formula (2) unless otherwise specified.

The value of $(X_2/Y) \times 100$ is not particularly limited as long as it is 1.6000 or less, and the smaller the value is, the more preferable it is. The value of $(X_2/Y) \times 100$ is preferably 1.5000 or less, more preferably 1.2000 or less, and still more preferably 1.0000 or less. The lower limit of $(X_2/Y) \times 100$ is a detection limit. In terms of obtaining an effect that matches an effort to decrease the value of $(X_2/Y) \times 100$ (e.g., removal and separation of the second peptide fragment by purification), the value of $(X_2/Y) \times 100$ is preferably 0.0500 or more, more preferably 0.0600 or more, and still more preferably 0.0700 or more.

The smaller the peak area value of the second peptide fragment calculated by an automatic integration method from an extracted ion chromatogram obtained by an LC-MS/MS analysis performed by using an aqueous solution prepared from the composition according to the first, second, third, fourth, fifth or sixth aspect and with an alkaline phosphatase concentration of 10% by weight is, the more preferable it is. The peak area value of the second peptide fragment is preferably 4,500 or less, more preferably 3,000 or less, and still more preferably 2,500 or less. The lower limit of the peak area value of the second peptide fragment is a detection limit. In terms of obtaining an effect that matches an effort to decrease the peak area value of the second peptide fragment (e.g., removal and separation of the second peptide fragment by purification), the peak area value of the second peptide fragment is preferably 100 or more, more preferably 150 or more, and still more preferably 200 or more.

The peak area value of an alkaline phosphatase calculated by an automatic integration method from a chromatogram obtained by an LC-UV analysis performed by using an aqueous solution prepared from the composition according to the first, second, third, fourth, fifth or sixth aspect and with an alkaline phosphatase concentration of 10% by weight is preferably 200,000 or more, more preferably 220,000 or more, and still more preferably 240,000 or more.

The upper limit of the peak area value of an alkaline phosphatase is not particularly limited. The peak area value of an alkaline phosphatase is preferably 500,000 or less, more preferably 400,000 or less, and still more preferably 350,000 or less.

[Content Ratio of Third Peptide Fragment to Alkaline Phosphatase]

In the composition according to the fifth aspect, the content ratio of the third peptide fragment to the alkaline phosphatase satisfies the following formula (3):

$$(X_3/Y) \times 100 \leq 0.2000 \qquad (3).$$

In an embodiment in which the composition according to the first, second, third, fourth or sixth aspect contains the third peptide fragment, the content ratio of the third peptide fragment to the alkaline phosphatase preferably satisfies the following formula (3):

$$(X_3/Y) \times 100 \leq 0.2000 \qquad (3).$$

In the formula (3), $X_3$ represents a peak area value of the third peptide fragment calculated by an automatic integration method from an extracted ion chromatogram obtained by an LC-MS/MS analysis of the composition according to the first, second, third, fourth, fifth or sixth aspect, and Y represents a peak area value of the alkaline phosphatase calculated by an automatic integration method from a chromatogram obtained by an LC-UV analysis of the composition according to the first, second, third, fourth, fifth or sixth aspect. The descriptions on the formula (A) (including the descriptions on the LC-MS/MS analysis and the LC-UV analysis) also apply to the formula (3) unless otherwise specified.
The value of $(X_3/Y) \times 100$ is not particularly limited as long as it is 0.2000 or less, and the smaller the value is, the more preferable it is. The value of $(X_3/Y) \times 100$ is preferably 0.1800 or less, more preferably 0.1700 or less, and still more preferably 0.1500 or less. The lower limit of $(X_3/Y) \times 100$ is a detection limit. In terms of obtaining an effect that matches an effort to decrease the value of $(X_3/Y) \times 100$ (e.g., removal and separation of the third peptide fragment by purification), the value of $(X_3/Y) \times 100$ is preferably 0.0500 or more, more preferably 0.0600 or more, and still more preferably 0.0700 or more.
The smaller the peak area value of the third peptide fragment calculated by an automatic integration method from an extracted ion chromatogram obtained by an LC-MS/MS analysis performed by using an aqueous solution prepared from the composition according to the first, second, third, fourth, fifth or sixth aspect and with an alkaline phosphatase concentration of 10% by weight is, the more preferable it is. The peak area value of the third peptide fragment is preferably 500 or less, more preferably 450 or less, and still more preferably 400 or less. The lower limit of the peak area value of the third peptide fragment is a detection limit. In terms of obtaining an effect that matches an effort to decrease the peak area value of the third peptide fragment (e.g., removal and separation of the third peptide fragment by purification), the peak area value of the third peptide fragment is preferably 100 or more, more preferably 150 or more, and still more preferably 200 or more.
The peak area value of an alkaline phosphatase calculated by an automatic integration method from a chromatogram obtained by an LC-UV analysis performed by using an aqueous solution prepared from the composition according to the first, second, third, fourth, fifth or sixth aspect and with an alkaline phosphatase concentration of 10% by weight is preferably 200,000 or more, more preferably 220,000 or more, and still more preferably 240,000 or more. The upper limit of the peak area value of an alkaline phosphatase is not particularly limited. The peak area value of an alkaline phosphatase is preferably 500,000 or less, more preferably 400,000 or less, and still more preferably 350,000 or less.

[Content Ratio of Fourth Peptide Fragment to Alkaline Phosphatase]

In the composition according to the sixth aspect, the content ratio of the fourth peptide fragment to the alkaline phosphatase satisfies the following formula (4):

$$(X_4/Y) \times 100 \leq 0.3500 \qquad (4).$$

In an embodiment in which the composition according to the first, second, third, fourth or fifth aspect contains the fourth peptide fragment, the content ratio of the fourth peptide fragment to the alkaline phosphatase preferably satisfies the following formula (4):

$$(X_4/Y) \times 100 \leq 0.3500 \qquad (4).$$

In the formula (4), $X_4$ represents a peak area value of the fourth peptide fragment calculated by an automatic integration method from an extracted ion chromatogram obtained by an LC-MS/MS analysis of the composition according to the first, second, third, fourth, fifth or sixth aspect, and Y represents a peak area value of the alkaline phosphatase calculated by an automatic integration method from a chromatogram obtained by an LC-UV analysis of the composition according to the first, second, third, fourth, fifth or sixth aspect. The descriptions on the formula (A) (including the descriptions on the LC-MS/MS analysis and the LC-UV analysis) also apply to the formula (4) unless otherwise specified.

The value of $(X_4/Y) \times 100$ is not particularly limited as long as it is 0.3500 or less, and the smaller the value is, the more preferable it is. The value of $(X_4/Y) \times 100$ is preferably 0.3200 or less, more preferably 0.3000 or less, and still more preferably 0.2800 or less. The lower limit of $(X_4/Y) \times 100$ is a detection limit. In terms of obtaining an effect that matches an effort to decrease the value of $(X_4/Y) \times 100$ (e.g., removal and separation of the fourth peptide fragment by purification), the value of $(X_4/Y) \times 100$ is preferably 0.0800 or more, more preferably 0.1000 or more, and still more preferably 0.1500 or more.

The smaller the peak area value of the fourth peptide fragment calculated by an automatic integration method from an extracted ion chromatogram obtained by an LC-MS/MS analysis performed by using an aqueous solution prepared from the composition according to the first, second, third, fourth, fifth or sixth aspect and with an alkaline phosphatase concentration of 10% by weight is, the more preferable it is. The peak area value of the fourth peptide fragment is preferably 1000 or less, more preferably 900 or less, and still more preferably 800 or less. The lower limit of the peak area value of the fourth peptide fragment is a detection limit. In terms of obtaining an effect that matches an effort to decrease the peak area value of the fourth peptide fragment (e.g., removal and separation of the fourth peptide fragment by purification), the peak area value of the fourth peptide fragment is preferably 100 or more, more preferably 150 or more, and still more preferably 200 or more.

The peak area value of an alkaline phosphatase calculated by an automatic integration method from a chromatogram obtained by an LC-UV analysis performed by using an aqueous solution prepared from the composition according to the first, second, third, fourth, fifth or sixth aspect and with an alkaline phosphatase concentration of 10% by weight is preferably 200,000 or more, more preferably 220,000 or more, and still more preferably 240,000 or more. The upper limit of the peak area value of an alkaline phosphatase is not particularly limited. The peak area value of an alkaline phosphatase is preferably 500,000 or less, more preferably 400,000 or less, and still more preferably 350,000 or less.

[Alkaline Phosphatase Specific Activity]

The composition of the present invention preferably has an alkaline phosphatase specific activity of 2,000 U/mg or more. The alkaline phosphatase specific activity of the composition of the present invention is more preferably 2,500 U/mg or more, and still more preferably 3,000 U/mg or more. The alkaline phosphatase specific activity of the composition of the present invention is measured as follows. By measuring the absorbance at 405 nm derived from p-nitrophenol produced by adding an alkaline phosphatase to an aqueous solution of p-nitrophenylphosphate, it is possible to calculate the specific activity of the alkaline phosphatase.

[Other Components]

The composition of the present invention can contain one or two or more other components. Examples of the other components include aqueous vehicles such as water, metal salts such as a magnesium salt and a sodium salt, surfactants, organic acids, glycerin and the like.

The composition of the present invention can be used for various applications requiring an alkaline phosphatase activity, and can contain one or two or more other components selected according to the applications.

In one embodiment, the composition of the present invention contains a protein such as an antigen and an antibody. In this embodiment, the composition of the present invention can be used for labeling a protein such as an antigen and an antibody with the alkaline phosphatase. In other words, in one embodiment, the composition of the present invention is a composition used for labeling a protein with the alkaline phosphatase. Labeling of a protein with the alkaline phosphatase can be performed by reacting a succinimide ester of the alkaline phosphatase, which is obtained by esterifying a carboxyl group of the alkaline phosphatase with succinimide, with an amino group of the protein.

In one embodiment, the composition of the present invention contains a substrate for the alkaline phosphatase. In this embodiment, the composition of the present invention can be used for dephosphorylating a substrate for the alkaline phosphatase. In other words, in one embodiment, the composition of the present invention is a composition used for dephosphorylating a substrate for the alkaline phosphatase. The substrate for the alkaline phosphatase is not particularly limited as long as the substrate is a compound having a phosphoric monoester bond. Examples of the substrate for the alkaline phosphatase include a nucleic acid, a phospholipid, pyrophosphoric acid and the like. When the substrate for the alkaline phosphatase is treated with the composition of the present invention, the phosphoric monoester bond of the substrate for the alkaline phosphatase is hydrolyzed by the alkaline phosphatase, resulting in dephosphorylation of the substrate for the alkaline phosphatase.

In a preferred embodiment, the composition of the present invention contains a nucleic acid. In this embodiment, the composition of the present invention can be used for dephosphorylating a nucleic acid. In other words, in a preferred embodiment, the composition of the present invention is a composition used for dephosphorylating a nucleic acid. The peptide fragment group (A), the peptide fragment group (B), the peptide fragment group (C), the second peptide fragment, the third peptide fragment or the fourth peptide fragment coexisting in the alkaline phosphatase has a possibility of adversely influencing when the nucleic acid is dephosphorylated by the alkaline phosphatase. In this regard, in the composition of the present invention, the content ratio of the peptide fragment group (A), the peptide fragment group (B), the peptide fragment group (C), the second peptide fragment, the third peptide fragment or the fourth peptide fragment to the alkaline phosphatase satisfies the above predetermined formula. In other words, in the composition of the present invention, the relative content of the peptide fragment group (A), the peptide fragment group (B), the peptide fragment group (C), the second peptide fragment, the third peptide fragment or the fourth peptide fragment has been reduced. Therefore, by using the composition of the present invention, it is possible to reduce the adverse influence of the peptide fragment group (A), the peptide fragment group (B), the peptide fragment group (C), the second peptide fragment, the third peptide fragment or the fourth peptide fragment that can occur when the nucleic acid is dephosphorylated by the alkaline phosphatase, thus enabling improvement in the dephosphorylation efficiency of the nucleic acid. By treating the nucleic acid with the composition of the present invention, the 5' end and/or the 3' end of the nucleic acid can be dephosphorylated. By dephosphorylating the 5' end and/or the 3' end of the nucleic acid, it is possible to improve the labeling efficiency when the 5' end and/or the 3' end of the nucleic acid is/are labeled with the labeling substance. Particularly, when $^{32}$P is used as the labeling substance, this effect is remarkable. By dephosphorylating the 5' end and/or the 3' end of a vector used for DNA cloning, self-ligation of the vector can be prevented, thus enabling a reduction in the background of a transformed cell.

Examples of the nucleic acid include nucleic acids such as DNA, RNA, peptide nucleic acid (PNA) and locked nucleic acid (LNA) or a nucleic acid derivative. Examples of the nucleic acid derivative include a nucleic acid derivative containing a modified nucleotide (e.g., a nucleotide that has undergone reconstitution of a nucleotide or base containing a halogen group, an alkyl group such as a methyl group, an alkoxy group such as a methoxy group, a thio group and a carboxymethyl group, etc., saturation of a double bond, deamination, substitution of an oxygen molecule with a sulfur molecule and the like). The nucleic acid may be single-stranded or double-stranded. Examples of the DNA include chromosomal DNA, viral DNA, DNA of a bacterium, a fungus, etc., cDNA, fragments thereof and the like. Examples of the RNA include mRNA, rRNA, small RNA, fragments thereof and the like. The nucleic acid may be chemically synthesized DNA, RNA and the like. Specific examples of the nucleic acid include a gene of a pathogen, a virus, etc., or a part thereof, a causative gene for genetic disease or a part thereof and the like.

The nucleic acid can be prepared by an extraction by a conventional method from, for example, a biomaterial, a virus, a bacterium, a fungus, a food and drink and the like. Examples of the biomaterial include body fluids such as blood, serum, plasma, urine, stool, spinal fluid, saliva, swab and tissue fluid, a cell, a tissue and the like. The biomaterial may be animal-derived or plant-derived.

The amount of the nucleic acid contained in the composition of the present invention can be appropriately adjusted according to the intended use of the composition of the present invention (e.g., detection of the target nucleic acid), etc. For example, when a certain nucleic acid (i.e., a target nucleic acid) among nucleic acids contained in the composition of the present invention is intended to be detected, it is possible to amplify the target nucleic acid by performing a nucleic acid amplification method, such as PCR, by using the nucleic acids contained in the composition of the present invention as a template. This enables improvement in the detection sensitivity of the target nucleic acid.

The length (number of bases) of the nucleic acid can be appropriately adjusted according to the intended use of the composition of the present invention (e.g., detection of the target nucleic acid), etc. For example, when the nucleic acid is intended to be detected, the length of the nucleic acid is usually 10 to 300 bases, preferably 10 to 100 bases, and more preferably 15 to 100 bases. The length of the nucleic acid can be appropriately adjusted by fragmentation treatment. The length of the nucleic acid is, for example, a length at which the nucleic acid can be hybridized with a probe. When the nucleic acid is long (e.g., 1,500 bases or more, particularly 4,000 bases or more), it is preferable to perform fragmentation treatment of the nucleic acid and to adjust the length of the nucleic acid to an appropriate length. When fragmentation treatment is performed, it is not necessarily that a specific nucleic acid fragment is selected from the generated nucleic acid fragments, and the fragmentation product can be used as it is.

Examples of a method for cleaving the nucleic acid for fragmentation include a method for cleaving by irradiation with ultrasonic waves, a method for cleaving with an enzyme, a method for cleaving with a restriction enzyme, a method using a nebulizer, a method for cleaving with an acid or an alkali and the like. In the case of the method for cleaving with ultrasonic waves, by controlling the output intensity and the irradiation time of the ultrasonic waves with which the nucleic acid is irradiated, it is possible to cleavage the nucleic acid into a desired length.

In a preferred embodiment, the composition of the present invention contains a dephosphorylated nucleic acid. The descriptions on the nucleic acid are the same as mentioned above. The dephosphorylated nucleic acid has a 5' end and/or a 3' end, each of which has been dephosphorylated by the alkaline phosphatase. In this embodiment, the composition of the present invention can be used for preparing a labeled nucleic acid containing the dephosphorylated nucleic acid and a labeling substance bound to the dephosphorylated nucleic acid. In other words, in a preferred embodiment, the composition of the present invention is a composition used for preparing a labeled nucleic acid containing the dephosphorylated nucleic acid and a labeling substance bound to the dephosphorylated nucleic acid. The peptide fragment group (A), the peptide fragment group (B), the peptide fragment group (C), the second peptide fragment, the third peptide fragment or the fourth peptide fragment coexisting in the alkaline phosphatase has a possibility of adversely influencing when the nucleic acid is dephosphorylated by the alkaline phosphatase and/or when the labeling substance is bound to the dephosphorylated nucleic acid. In this regard, in the composition of the present invention, the content ratio of the peptide fragment group (A), the peptide fragment group (B), the peptide fragment group (C), the second peptide fragment, the third peptide fragment or the fourth peptide fragment to the alkaline phosphatase satisfies the above predetermined formula. In other words, in the composition of the present invention, the relative contents of the peptide fragment group (A), the peptide fragment group (B), the peptide fragment group (C), the second peptide fragment, the third peptide fragment or the fourth peptide fragment has been reduced. Therefore, by using the composition of the present invention, it is possible to reduce the adverse influence of the peptide fragment group (A), the peptide fragment group (B), the peptide fragment group (C), the second peptide fragment, the third peptide fragment or the fourth peptide fragment that can occur when the nucleic acid is dephosphorylated by the alkaline phosphatase and/or when the labeling substance is bound to the dephosphorylated nucleic acid, thus enabling improvement in the dephosphorylation efficiency of the nucleic acid and/or the labeling efficiency of the dephosphorylated nucleic acid.

In a preferred embodiment, the composition of the present invention contains a labeled nucleic acid containing a dephosphorylated nucleic acid and a labeling substance bound to the dephosphorylated nucleic acid. The descriptions on the nucleic acid are the same as mentioned above. The dephosphorylated nucleic acid has a 5' end and/or a 3' end, each of which has been dephosphorylated by the alkaline phosphatase. The labeling substance is bound to the 5' end and/or the 3' end of the dephosphorylated nucleic acid.

As the labeling substance, for example, a fluorescent dye, a fluorescent protein, a chemiluminescent body, a metal complex, a metal fine particle, biotin, a radioisotope, etc., can be used. The reaction conditions when the target nucleic acid is labeled can be appropriately adjusted according to the type of the labeling substance. When the labeling substance is a fluorescent dye, the fluorescent dye can be detected by using a fluorescence microscope, a fluorescence scanner and the like.

Examples of the fluorescent dye include organic fluorescent dyes such as a fluorescein-based dye, a rhodamine-based dye, an Alexa Fluor (manufactured by Invitrogen)-based dye, a BODIPY (manufactured by Invitrogen)-based dye, a cascade-based dye, a coumarin-based dye, an eosin-based dye, an NBD-based dye, a pyrene-based dye, a Texas Red-based dye and a cyanine-based dye.

Specific examples of the organic fluorescent dye include
5-carboxy-fluorescein, 6-carboxy-fluorescein,
5,6-dicarboxy-fluorescein,
6-carboxy-2',4,4',5',7,7'-hexachloro-fluorescein,
6-carboxy-2',4,7,7'-tetrachloro-fluorescein,
6-carboxy-4',5'-dichloro-2',7'-dimethoxy-fluorescein,
naphtho-fluorescein, 5-carboxy-rhodamine, 6-carboxy-rhodamine, 5,6-dicarboxy-rhodamine, rhodamine 6G, tetramethylrhodamine, X-rhodamine, Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 635, Alexa Fluor 647, Alexa Fluor 660, Alexa Fluor 680, Alexa Fluor 700, Alexa Fluor 750, BODIPY FL, BODIPY TMR, BODIPY 493/503, BODIPY 530/550, BODIPY 558/568, BODIPY 564/570, BODIPY 576/589, BODIPY 581/591, BODIPY 630/650, BODIPY 650/665 (all of which are manufactured by Invitrogen), methoxycoumarin, eosin, NBD, pyrene, Cy5, Cy5.5, Cy7 and the like.

In the embodiment in which the composition of the present invention contains a labeled nucleic acid containing a dephosphorylated nucleic acid and a labeling substance bound to the dephosphorylated nucleic acid, the composition of the present invention can be used as a nucleic acid sample to be subjected to a nucleic acid detection method. In other words, in a preferred embodiment, the composition of the present invention is a nucleic acid sample to be subjected to the nucleic acid detection method. The labeled nucleic acid can contain a target nucleic acid to be detected and a nucleic acid other than the target nucleic acid. In the nucleic acid detection method, the target nucleic acid contained in the nucleic acid sample can be detected by using the labeling substance of the target nucleic acid as an index. The nucleic acid detection method is not particularly limited, and can be appropriately selected from known nucleic acid detection methods. The target nucleic acid can be detected, for example, by using the hybridization method. In one embodiment of the hybridization method, the target nucleic acid can be detected by using a probe that can be hybridized with the target nucleic acid. In one embodiment of the nucleic acid detection method using a probe, the probe is brought into contact with the nucleic acid sample containing the target nucleic acid to hybridize the probe with the target nucleic acid, and the target nucleic acid hybridized with the probe can be detected by using the labeling substance of the target nucleic acid as an index. When a nucleic acid other than the target nucleic acid is contained in the sample, it is preferable that, after the target nucleic acid is brought into contact with the probe, the nucleic acid that was not hybridized with the probe is removed by washing, etc.

The reaction conditions when the target nucleic acid is hybridized with the probe can be appropriately adjusted according to chain length of the target nucleic acid, the chain length of the probe and the like. The reaction time is usually 30 to 1,200 minutes, and preferably 60 to 360 minutes. The reaction temperature is usually 25 to 60°C, and preferably 30 to 40°C. The reaction is usually performed in an aqueous vehicle such as water.

The amount of the target nucleic acid or probe used is not particularly limited as long as the hybridization between the target nucleic acid and the probe can occur and the labeling substance bound to the target nucleic acid can be detected, and the amount can be appropriately adjusted according to the chain length of the target nucleic acid, the chain length of the probe, the type of the labeling substance and the like.

As the probe, for example, nucleic acids such as DNA, RNA, peptide nucleic acid (PNA) and locked nucleic acid (LNA) or a nucleic acid derivative can be used. Examples of the nucleic acid derivative include a nucleic acid derivative containing a modified nucleotide (e.g., a nucleotide that has undergone reconstitution of a nucleotide or base containing a halogen group, an alkyl group such as a methyl group, an alkoxy group such as a methoxy group, a thio group and a carboxymethyl group, etc., saturation of a double bond, deamination, substitution of an oxygen molecule with a sulfur molecule and the like).

The probe has a base sequence complementary to at least a part of the base sequence of the target nucleic acid, and can be hybridized with the target nucleic acid. When the target nucleic acid is double-stranded, the probe may be hybridized with a sense strand or may be hybridized with an antisense strand. The base sequence of the probe may be complementary to any part of the base sequence of the target nucleic acid, and is preferably complementary to a highly-specific part of the base sequence of the target nucleic acid. In other words, the base sequence of the probe is preferably complementary to a base sequence which other nucleic acids contained in the sample do not have, of the base sequence of the target nucleic acid.

Of the base sequence of the probe, the length (number of bases) of the part complementary to the base sequence of the target nucleic acid is not particularly limited, and is usually 10 to 150 bases, preferably 20 to 100 bases, and more preferably 20 to 70 bases. The probe may be composed of a base sequence complementary to the base sequence of the target nucleic acid, or may include a base sequence not complementary to the base sequence of the target nucleic acid. The full length (total number of bases) of the probe is usually 10 to 300 bases, preferably 20 to 200 bases, and more preferably 15 to 100 bases.

The probe may be any of a commercially available product, a synthetic product, a prepared product from a living body and the like. A nucleic acid having a length of up to 200 bases, which is referred to as an oligonucleic acid, can be easily artificially synthesized with a synthesizer.

The probe is preferably fixed to a support. In other words, in a preferred embodiment, the nucleic acid detection method is a nucleic acid detection method using a nucleic acid microarray. The nucleic acid microarray has a support and a plurality of probes fixed to the surface of the support. In the nucleic acid detection method using a nucleic acid microarray, the labeled target nucleic acid is brought into contact with a nucleic acid microarray provided with a probe that can be hybridized with the target nucleic acid, and the target nucleic acid hybridized with the probe can be detected by using the labeling substance bound to the target nucleic acid as an index. When a nucleic acid other than the target nucleic acid is contained in the sample, it is preferable that, after the target nucleic acid is brought into contact with the nucleic acid microarray, the nucleic acid that has not been hybridized with the probe on the nucleic acid microarray is removed by washing, etc. By using a nucleic acid microarray provided with a plurality of probes, two or more target nucleic acids can be simultaneously detected.

The support is not particularly limited as long as it can fix the probe. Examples of the support include a slide, a membrane, a bead and the like. Examples of the material of the support include inorganic materials such as glass, ceramic and silicon, and polymers such as polyethylene terephthalate, cellulose acetate, polycarbonate, polystyrene, polymethyl methacrylate and silicone rubber and the like.

Fixation of the probe to the support can be performed in accordance with a conventional method. As a method for fixing the probe to the support, a method for synthesizing an oligonucleic acid on the top surface of the support, a method for adding dropwise an oligonucleic acid synthesized in advance to the top surface of the support to fix, etc., are known. Examples of the former method include the method by Ronald et al. (US 5705610 A), the method by Michel et al. (US 6142266 A), the method by Francesco et al. (US 7037659 A) and the like. In these methods, since an organic solvent is used during DNA synthesis reaction, the support is desirably a material that is resistance to an organic solvent. For example, it is possible to use a glass support having an irregular structure fabricated by using the method disclosed in JP H10-503841 A. Particularly, in the method by Francesco et al., since the back of the support is irradiated with light to control DNA synthesis, the support is preferably a material having translucency. Examples of the latter method include the method by Hirota et al. (JP 3922454 B2), a method using a glass capillary and the like. As an example of the glass capillary, it is possible to use a self-made glass capillary, commercially available products such as a micropipette (manufactured by Micro Support Co., Ltd.; MP-005) and the like.

As a method for detecting the target nucleic acid, the sandwich hybridization method can be used. In the sandwich hybridization method, a first probe (capture probe) fixed to the support and a second probe (detection probe) not fixed to the support are used. The capture probe and the detection probe each have a base sequence complementary to different parts of the target nucleic acid, and can be hybridized with different parts of the target nucleic acid. The target nucleic acid is hybridized with the detection probe and the capture probe, thus forming a complex. By detecting a labeling substance contained in this complex, the target nucleic acid can be detected.

The sequence identity between the base sequence of the detection probe and the base sequence of the capture probe is preferably low. The sequence identity is preferably 20% or less, and more preferably 10% or less. In this regard, the identity between two base sequences is a numerical value obtained by aligning two sequences (inserting a gap, if necessary) so that bases are matched as many as possible, and then by dividing the number of matched bases by total number of bases (the higher number of bases when the number of bases of two base sequences is different), and the identity can be easily calculated with commercially available software such as FASTA and BLAST (also provided via the internet).

The signal detected in the method for detecting the target nucleic acid (e.g., intensity of the detected labeling substance) is compared with a surrounding noise. Specifically, the signal value obtained from a position on the support at which a probe is fixed is compared with the signal value (noise value) obtained from a position of the support at which no probe is fixed, and a ratio of the former numerical value to the noise value is defined as an S/N ratio. The detection accuracy can be represented by the S/N ratio. In other words, the larger the S/N ratio is, the higher the detection accuracy is, and the smaller the S/N ratio is, the lower the detection accuracy is.

In the embodiment in which the composition of the present invention contains a labeled nucleic acid containing a dephosphorylated nucleic acid and a labeling substance bound to the dephosphorylated nucleic acid, by using the composition of the present invention as a nucleic acid sample in the nucleic acid detection method, it is possible to improve the detection sensitivity of the target nucleic acid. This effect is remarkable in a nucleic acid detection method using an extremely small amount (preferably 5 to 1,000 μL, more preferably 5 to 500 μL) of a nucleic acid sample. In the nucleic acid detection method using an extremely small amount of a nucleic acid sample, the peptide fragment group (A), the peptide fragment group (B), the peptide fragment group (C), the second peptide fragment, the third peptide fragment or the fourth peptide fragment contained in the nucleic acid sample has a possibility of adversely influencing the detection sensitivity. In this regard, in the composition of the present invention, the content ratio of the peptide fragment group (A), the peptide fragment group (B), the peptide fragment group (C), the second peptide fragment, the third peptide fragment or the fourth peptide fragment to the alkaline phosphatase satisfies the above predetermined formula. In other words, in the composition of the present invention, the relative content of the peptide fragment group (A), the peptide fragment group (B), the peptide fragment group (C), the second peptide fragment, the third peptide fragment or the fourth peptide fragment has been reduced. Therefore, in the nucleic acid detection method using an extremely small amount of a nucleic acid sample, by using the composition of the present invention as the nucleic acid sample, it is possible to reduce the adverse influence of the peptide fragment group (A), the peptide fragment group (B), the peptide fragment group (C), the second peptide fragment, the third peptide fragment or the fourth peptide fragment, thus enabling remarkable improvement in the detection sensitivity of the target nucleic acid.

In a preferred embodiment, the nucleic acid detection method is a nucleic acid detection method using a nucleic acid microarray. In the nucleic acid detection method using a nucleic acid microarray, an extremely small amount (preferably 5 to 1,000 μL, more preferably 5 to 500 μL) of a nucleic acid sample is used. Therefore, in the nucleic acid detection method using a nucleic acid microarray, by using the composition of the present invention as the nucleic acid sample, it is possible to reduce the adverse influence of the peptide fragment group (A), the peptide fragment group (B), the peptide fragment group (C), the second peptide fragment, the third peptide fragment or the fourth peptide fragment, thus enabling remarkable improvement in the detection sensitivity of the target nucleic acid.

[Production Method]

The composition of the present invention can be produced by separating the peptide fragment group (A), the peptide fragment group (B), the peptide fragment group (C), the second peptide fragment, the third peptide fragment or the fourth peptide fragment from an alkaline phosphatase extract from an organ of a bovine, a shrimp, etc., an alkaline phosphatase extract from a microorganism into which a gene encoding an alkaline phosphatase has been introduced, a bacterial cell homogenate of a microorganism into which a gene encoding an alkaline phosphatase has been introduced, a commercially available alkaline phosphatase product and the like. Examples of the method for separating the peptide fragment group (A), the peptide fragment group (B), the peptide fragment group (C), the second peptide fragment, the third peptide fragment or the fourth peptide fragment include dialysis, salting out, gel filtration, ultrafiltration, membrane separation, ion exchange, column chromatography, electrophoresis and the like. Regarding the method for separating the peptide fragment, one separation method may be used alone, or two or more separation methods may be used in combination. For example, by purifying a commercially available alkaline phosphatase product by column chromatography, etc., it is possible to make the content of the peptide fragment group (A), the peptide fragment group (B), the peptide fragment group (C), the second peptide fragment, the third peptide fragment or the fourth peptide fragment to the alkaline phosphatase be to a desired range. The column chromatography is, for example, liquid chromatography. The column and the mobile phase used in liquid chromatography is not particularly limited as long as the peptide fragment group (A), the peptide fragment group (B), the peptide fragment group (C), the second peptide fragment, the third peptide fragment or the fourth peptide fragment can be separated, and it is preferable to use a C18-supported reverse-phase column.

[Use method]

The composition of the present invention can be used for various methods requiring an alkaline phosphatase activity.

In one embodiment, the composition of the present invention is used for a method for producing a dephosphorylated nucleic acid, the method including the following steps of:

providing the composition of the present invention;
providing a nucleic acid; and
treating the nucleic acid with the composition to dephosphorylate the nucleic acid. The peptide fragment group (A), the peptide fragment group (B), the peptide fragment group (C), the second peptide fragment, the third peptide fragment or the fourth peptide fragment coexisting in the alkaline phosphatase has a possibility of adversely influencing when the nucleic acid is dephosphorylated by the alkaline phosphatase. In this regard, in the composition of the present invention, the content ratio of the peptide fragment group (A), the peptide fragment group (B), the peptide fragment group (C), the second peptide fragment, the third peptide fragment or the fourth peptide fragment to the alkaline phosphatase satisfies the above predetermined formula. In other words, in the composition of the present invention, the relative content of the peptide fragment group (A), the peptide fragment group (B), the peptide fragment group (C), the second peptide fragment, the third peptide fragment or the fourth peptide fragment has been reduced. Thus, by treating the nucleic acid with the composition of the present invention, it is possible to improve the dephosphorylation efficiency of the nucleic acid.

In one embodiment, the composition of the present invention is used for a method for producing a labeled nucleic acid, the method including the following steps of:

providing the composition of the present invention;
providing a nucleic acid;
providing a labeling substance;
treating the nucleic acid with the composition to dephosphorylate the nucleic acid; and
binding the labeling substance to the dephosphorylated nucleic acid. The peptide fragment group (A), the peptide fragment group (B), the peptide fragment group (C), the second peptide fragment, the third peptide fragment or the fourth peptide fragment coexisting in the alkaline phosphatase has a possibility of adversely influencing when the nucleic acid is dephosphorylated by the alkaline phosphatase and/or when the labeling substance is bound to the dephosphorylated nucleic acid. In this regard, in the composition of the present invention, the content ratio of the peptide fragment group (A), the peptide fragment group (B), the peptide fragment group (C), the second peptide fragment, the third peptide fragment or the fourth peptide fragment to the alkaline phosphatase satisfies the above predetermined formula. In other words, in the composition of the present invention, the relative content of the peptide fragment group (A), the peptide fragment group (B), the peptide fragment group (C), the second peptide fragment, the third peptide fragment or the fourth peptide fragment has been reduced. Thus, by treating the nucleic acid with the composition of the present invention, it is possible to improve the dephosphorylation efficiency of the nucleic acid and/or the labeling efficiency of the dephosphorylated nucleic acid.

In the step of treating the nucleic acid with the composition of the present invention to dephosphorylate the nucleic acid, the reaction conditions can be appropriately adjusted. The reaction time is usually 10 to 60 minutes, and preferably 20 to 50 minutes. The reaction temperature is usually 20 to 60°C, and preferably 25 to 45°C. The reaction is usually performed in an aqueous vehicle such as water. The nucleic acid is, for example, DNA, RNA and the like. When the nucleic acid is treated with the composition of the present invention, the 5' end and/or the 3' end of the nucleic acid is dephosphorylated.

In the step of binding the labeling substance to the dephosphorylated nucleic acid, as the labeling substance, for example, a fluorescent dye, a fluorescent protein, a chemiluminescent body, a metal complex, a metal fine particle, biotin, a radioisotope, etc., can be used. The reaction conditions can be appropriately adjusted according to the type of the labeling substance. The dephosphorylated nucleic acid has a 5' end and/or a 3' end, each of which has been dephosphorylated by the alkaline phosphatase, and the labeling substance can be bound to the dephosphorylated 5' end and/or 3' end.

EXAMPLES

The present invention will be described in detail by way of Examples, but the present invention is not limited to the following Examples.

<Conditions of LC-MS/MS Analysis>

Conditions of the LC-MS/MS analysis used in Examples and Comparative Examples were as follows.

<Apparatus Configuration>

Mass spectrometer: maXis impact (manufactured by Bruker Daltnics, Inc.)

<Conditions of Mass Spectrometry>

Ionization method: ESI
Measured ion: cation
Capillary voltage: 4,500 V
Nebulizer: 2.0 bar
Dry gas: 8.0 L/min
Detector voltage: 1,823 V
Measuring span (MS): m/z 50 to 2,200

<MS/MS Conditions>

Measuring span (MS): m/z 50 to 2,200
Collision gas: nitrogen

<Conditions of LC-UV Analysis>

Conditions of the LC-UV analysis used in Examples and Comparative Examples were as follows.

<Apparatus Configuration>

Liquid chromatograph: LC-30A system (manufactured by Shimadzu Corporation)
Detector: UV-Vis (190 to 900 nm, manufactured by Shimadzu Corporation)

<Conditions of Liquid Chromatography>

Column: Acquity BEH C18 1.7 $\mu$m (manufactured by Waters Corporation)

Column size: 2.1 mm $\times$ 100 mm

Column temperature: 50°C
Mobile phase flow rate: 0.2 mL/min
Mobile phase A: mixed solution of water/formic acid (1000:1)
Mobile phase B: mixed solution of acetonitrile/water/formic acid (900:100:1)
Injection volume: 20 μL
Gradient program:

[Table 2]

| Times (min) | Mobile phase A (vol%) | Mobile phase B (vol%) |
|---|---|---|
| 0 | 100 | 0 |
| 10 | 100 | 0 |
| 40 | 35 | 65 |
| 40.1 | 0 | 100 |
| 50 | 0 | 100 |
| 50.1 | 100 | 0 |
| 60 | 100 | 0 |

<Nucleic Acid Detection Method>

The nucleic acid detection method used in Examples and Comparative Examples was as follows.
The detection method of a nucleic acid was performed by using a DNA chip (DNA microarray). Specifically, detection was performed by using "3D-Gene" human miRNA oligo chip (compatible with miRBase release 21) manufactured by Toray Industries, Inc.

[Comparative Examples 1 to 8]

Eight alkaline phosphatase products purchased from five companies (hereinafter referred to as "composition C1" to "composition C8") were used as the alkaline phosphatase compositions of Comparative Examples 1 to 8. The alkaline phosphatase contained in each of the compositions C1 to C8 was an alkaline phosphatase derived from the intestinal tract of a bovine. When the alkaline phosphatase specific activities of the compositions C1 to C8 were measured, they were 2,238 U/mg for the composition C1, 2,492 U/mg for the composition C2, 2,431 U/mg for the composition C3, 2,519 U/mg for the composition C4, 2,411 U/mg for the composition C5, 2,552 U/mg for the composition C6, 2,448 U/mg for the composition C7, and 2,490 U/mg for the composition C8. The alkaline phosphatase specific activities were measured by a method using p-nitrophenylphosphate. Specifically, the method was as follows.
The following solutions A and B were provided.

Solution A: 1M diethanolamine buffer (pH 9.8)
Solution B: aqueous 0.67M p-nitrophenolphosphate solution
2.9 mL of the solution A and 0.1 mL of the solution B were prepared in a cuvette (optical path length = 1 cm), and warmed at 37°C for 5 minutes. Then, 0.1 mL of an aqueous alkaline phosphatase solution was added, and the absorbance change at 405 nm (37°C) was measured with a spectrophotometer for 3 to 5 minutes to obtain an absorbance change per unit time (ΔOD). By using as a control, a sample to which water was added in place of the aqueous alkaline phosphatase solution, the absorbance change was obtained (ΔOD blank). The alkaline phosphatase activity (U/mL) was calculated from the following formula:

$$\text{Alkaline phosphatase activity (U/mL)} = (\Delta OD - \Delta OD \text{ blank}) \times 3.1/(18.2 \times 0.1 \times 1.0).$$

The concentration of the alkaline phosphatase in the aqueous alkaline phosphatase solution was calculated by measuring the absorbance at 214 nm. The aqueous alkaline phosphatase solution was diluted with distilled water so that the absorbance at 214 nm became 0.1 to 1.0, and 1 Abs was approximated to 1 mg/mL, and then the value obtained by multiplying by the dilution rate was regarded as the concentration of the alkaline phosphatase. The specific activity in the present invention represents an activity (U/mg) per 1 mg of the alkaline phosphatase, and was calculated by the abovementioned measurement method.

An aqueous 10% by weight alkaline phosphatase solution was prepared from each of the compositions C1 to C8, and by using this aqueous solution, an LC-UV analysis and an LC-MS/MS analysis were performed. Based on the extracted ion chromatogram obtained by the LC-MS/MS analysis, the peak area value of each of the first peptide fragment consisting of the amino acid sequence set forth in SEQ ID NO: 1 (VPLASETHGGEDVAVF), the second peptide fragment consisting of the amino acid sequence set forth in SEQ ID NO: 2 (VPLASETHGGEDV), the third peptide fragment consisting of the amino acid sequence set forth in SEQ ID NO: 3 (GPQAHLVHGVQEETFVAH) and the fourth peptide fragment consisting of the amino acid sequence set forth in SEQ ID NO: 4 (GPQAHLVHGVQE) was calculated by an automatic integration method. Based on the chromatogram obtained by the LC-UV analysis, the peak area value of the alkaline phosphatase was calculated by an automatic integration method. In the LC-UV analysis, the alkaline phosphatase was detected as a component having absorption at 214 nm.

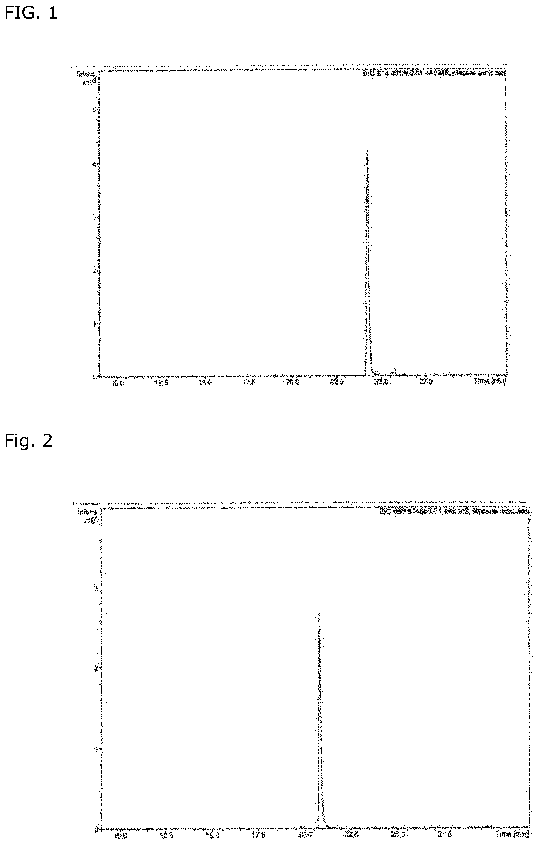

FIG. 1 shows an extracted ion chromatogram on the first peptide fragment obtained by an LC-MS/MS analysis of the composition C2 in Comparative Example 2.

FIG. 2 shows an extracted ion chromatogram on the second peptide fragment obtained by an LC-MS/MS analysis of the composition C2 in Comparative Example 2.

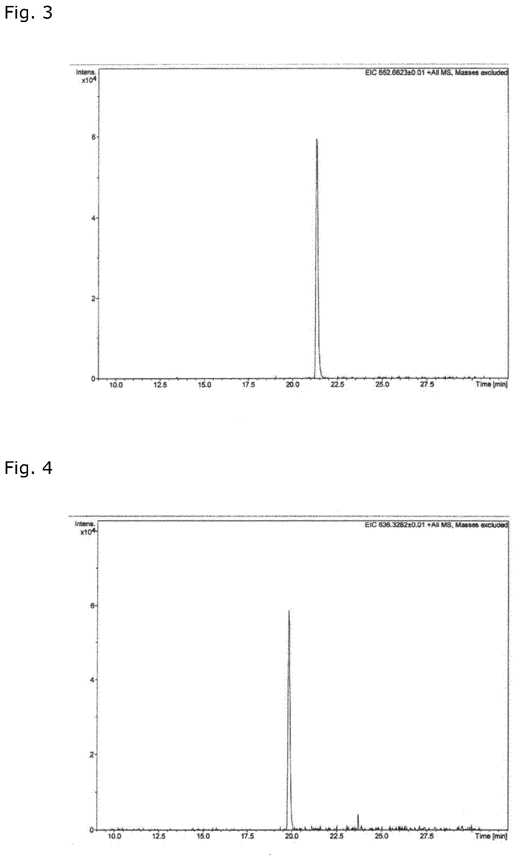

FIG. 3 shows an extracted ion chromatogram on the third peptide fragment obtained by an LC-MS/MS analysis of the composition C2 in Comparative Example 2.

FIG. 4 shows an extracted ion chromatogram on the fourth peptide fragment obtained by an LC-MS/MS analysis of the composition C2 in Comparative Example 2.

By using each of the alkaline phosphatase compositions of Comparative Examples 1 to 8, a nucleic acid was dephosphorylated, and the obtained dephosphorylated nucleic acid was labeled with a cyanine-based organic fluorescent dye. Specifically, dephosphorylation reaction and labeling reaction were performed as follows.

Whole blood collected from a healthy individual was centrifuged to obtain 1 mL of serum. From the serum, microRNA was extracted by using the "3D-Gene" RNA extraction reagent from liquid sample kit (manufactured by Toray Industries, Inc.). The obtained extracted microRNA was regarded as a mother liquor and was labeled by using "3D-Gene" miRNA labeling kit (manufactured by Toray Industries, Inc.). Specifically, 5 μL of the obtained extracted microRNA was added to a mixed solution of 0.4 μL of AP buffer and 1.0 μL of Spike Control of the abovementioned kit, and 0.4 μL of the composition C1 was further added to prepare a solution. Then, the prepared solution was incubated at 37°C for 40 minutes, followed by allowing to stand on ice for 2 minutes. Then, 1.2 μL of LE Buffer, 3.0 μL of 3D-Gene Fluorescent Label, 2.5 μL of Nuclease free water and 1.0 μL of Labeling enzyme were added, and the obtained solution was incubated at 16°C for 1 hour, followed by incubation at 65°C for 15 minutes to obtain a labeled nucleic acid. Dephosphorylation reaction and labeling reaction were performed by using the same method as mentioned above, in which the compositions C2 to C8 and the same extracted microRNA mother liquor were used.

By using the obtained labeled nucleic acid, detection of a nucleic acid was performed. Specifically, for the labeled sample RNA, hybridization was performed by using a DNA chip ("3D-Gene" miRNA chip, manufactured by Toray Industries, Inc.) in accordance with the standard protocol thereof. The DNA chip after hybridization was subjected to a microarray scanner (manufactured by Toray Industries, Inc.) to measure the fluorescence intensity. Regarding the setting of the scanner, the laser output was set at 100%, and the voltage setting of the photomultiplier was set at AUTO setting. Detection of a nucleic acid was performed by using a DNA chip (DNA microarray) as mentioned above. The number of valid spots in the DNA chip was determined to calculate the detection rate (%). Specifically, of a total of 2,588 spots on the DNA chip, spots with a value obtained by subtracting the noise (signal value at a site having no spot) from the detection signal value being 100 or more were regarded as valid spots, and the value obtained by dividing the number of valid spots by the number of all spots and by multiplying by 100 was regarded as the detection rate. The results are shown in Table 4-2.

[Examples 1 to 4]

The alkaline phosphatase compositions of Comparative Examples 2 to 4 and 8 (compositions C2 to C4 and C8) were purified by the following method to obtain alkaline phosphatase compositions of Examples 1 to 4 (hereinafter referred to as "composition E1" to "composition E4"). The purification method was as follows.

(Dialysis Step)

The composition C2 (30 μL) was dialyzed three times with a dialysis buffer (1 mL, 50 mM Tris-HCl, 2 mM MgCl$_2$, 0.2 mM ZnCl$_2$) by using a dialysis cup (cutoff molecular weight of 3.5 K), and the concentrate was collected.

(Gel Filtration Step)

The concentrate after dialysis treatment was collected by filtration with a buffer (2.5 mL, 10 mM Tris-HCl, 1 mM MgCl$_2$, 0.1 mM ZnCl$_2$, 50 mM KCl, 55% by weight glycerin) by using a gel filtration column.

(Hydrophobic Column Step)

From the collected solution after gel filtration, the alkaline phosphatase fraction was collected by using a hydrophobic column under the following conditions.

Mobile phase flow rate: 1.0 mL/min
Mobile phase A: 20 mM disodium hydrogenphosphate, 3M ammonium sulfate (50/50)
Mobile phase B: 20 mM disodium hydrogenphosphate Detector: UV 214 nm
Gradient program:

[Table 3]

| Times (min) | Mobile phase A (vol%) | Mobile phase B (vol%) |
|---|---|---|
| 0 | 100 | 0 |
| 3 | 100 | 0 |
| 40 | 0 | 100 |
| 50 | 0 | 100 |
| 55 | 100 | 0 |
| 65 | 100 | 0 |

(Dialysis Step)

The collected alkaline phosphatase fraction was dialyzed three times under the same conditions as for the abovementioned dialysis, and the concentrate was collected.

(Ultrafiltration Step)

The collected concentrate was collected by filtration with a buffer (2.5 mL, 10 mM Tris-HCl, 1 mM MgCl$_2$, 0.1 mM ZnCl$_2$, 50 mM KCl, 55% by weight glycerin) by using an ultrafiltration column (cutoff molecular weight of 10 K) to obtain the composition E1.

The compositions E2, E3 and E4 were also obtained from the compositions C3, C4 and C8, respectively, by using the same method as mentioned above.

When the alkaline phosphatase specific activities of the alkaline phosphatase compositions of Examples 1 to 4 were measured, they were 2,490 U/mg for the composition E1, 2,420 U/mg for the composition E2, 2,522 U/mg for the composition E3, and 2,470 U/mg for the composition E4. The alkaline phosphatase specific activities were measured in the same manner as mentioned above.

An aqueous 10% by weight alkaline phosphatase solution was prepared from each of the compositions E1 to E4, and by using this aqueous solution, an LC-UV analysis and an LC-MS/MS analysis were performed. Based on the extracted ion chromatogram obtained by the LC-MS/MS analysis, the peak area value of each of the first peptide fragment consisting of the amino acid sequence set forth in SEQ ID NO: 1 (VPLASETHGGEDVAVF), the second peptide fragment consisting of the amino acid sequence set forth in SEQ ID NO: 2 (VPLASETHGGEDV), the third peptide fragment consisting of the amino acid sequence set forth in SEQ ID NO: 3 (GPQAHLVHGVQEETFVAH) and the fourth peptide fragment consisting of the amino acid sequence set forth in SEQ ID NO: 4 (GPQAHLVHGVQE) was calculated by an automatic integration method. Based on the chromatogram obtained by the LC-UV analysis, the peak area value of the alkaline phosphatase was calculated by an automatic integration method. In the LC-UV analysis, the alkaline phosphatase was detected as a component having absorption at 214 nm.

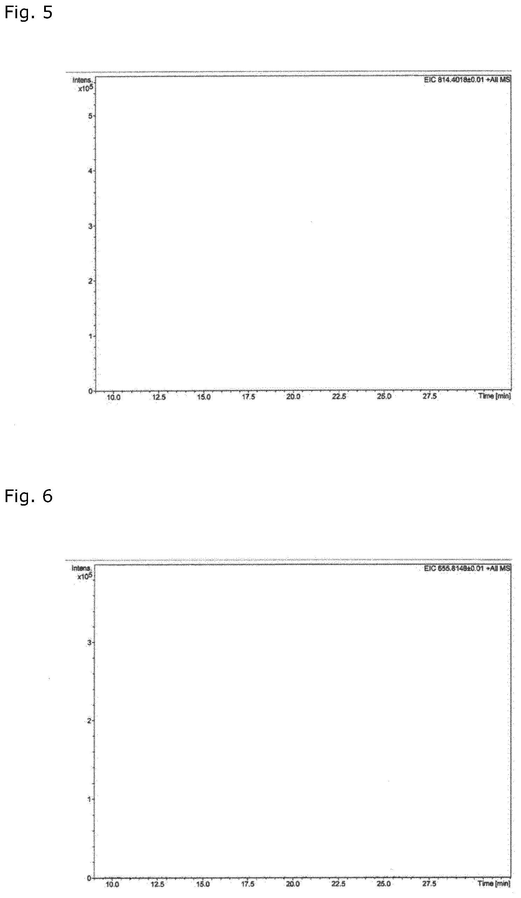

FIG. 5 shows an extracted ion chromatogram on the first peptide fragment obtained by an LC-MS/MS analysis of the composition E1 (purified product of the composition C2) in Example 1.

FIG. 6 shows an extracted ion chromatogram on the second peptide fragment obtained by an LC-MS/MS analysis of the composition E1 (purified product of the composition C2) in Example 1.

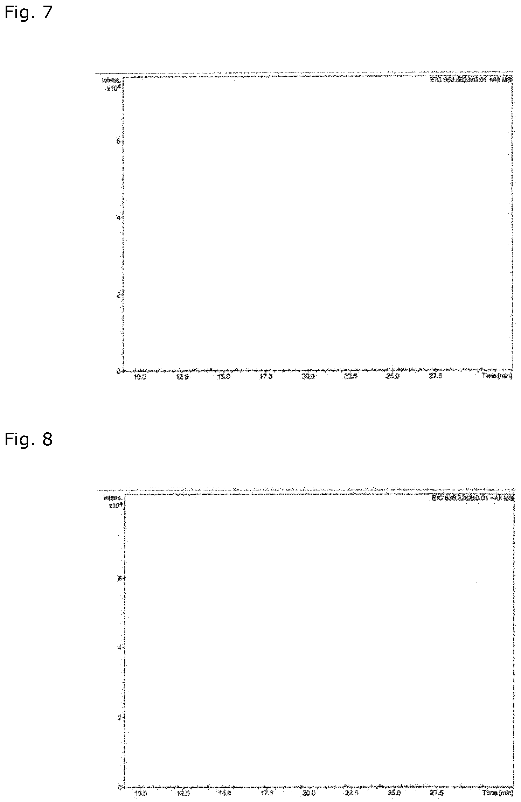

FIG. 7 shows an extracted ion chromatogram on the third peptide fragment obtained by an LC-MS/MS analysis of the composition E1 (purified product of the composition C2) in Example 1.

FIG. 8 shows an extracted ion chromatogram on the fourth peptide fragment obtained by an LC-MS/MS analysis of the composition E1 (purified product of the composition C2) in Example 1.

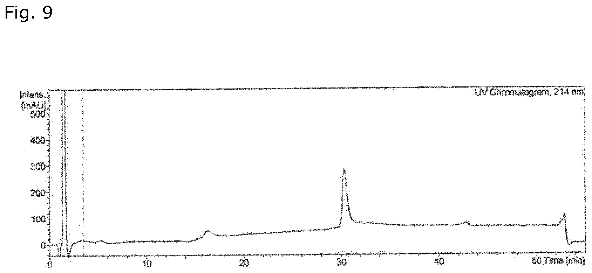

FIG. 9 shows a chromatogram on an alkaline phosphatase obtained by an LC-UV analysis of the composition E1 (purified product of the composition C2) in Example 1. It is noted that a chromatogram on an alkaline phosphatase obtained by an LC-UV analysis of each of the composition in Examples 2 to 4 and Comparative Examples 1 to 8 was the same as FIG. 9.

By using the alkaline phosphatase compositions of Examples 1 to 4, a nucleic acid was dephosphorylated, and the obtained dephosphorylated nucleic acid was labeled with a cyanine-based organic fluorescent dye. Dephosphorylation reaction and labeling reaction were performed in the same manner as mentioned above.

By using the obtained labeled nucleic acid, detection of a nucleic acid was performed. Detection of a nucleic acid was performed by using a DNA chip (DNA microarray) as mentioned above. The number of valid spots in the DNA chip was determined to calculate the detection rate (%). The results are shown in Table 4-1.

[Table 4-1]

|  | Examples | | | |
|---|---|---|---|---|
|  | 1 | 2 | 3 | 4 |
| Peak area value of First peptide fragment ($X_1$) | 1783 | 517 | 2416 | 246 |
| Peak area value of Second peptide fragment ($X_2$) | 1766 | 949 | 1769 | 585 |
| Peak area value of Third peptide fragment ($X_3$) | 226 | 226 | 360 | 200 |
| Peak area value of Fourth peptide fragment ($X_4$) | 668 | 367 | 736 | 278 |
| Peak area value of Alkaline phosphatase (Y) | 263754 | 268264 | 267135 | 258635 |
| $(X_1/Y) \times 100$ | 0.6762 | 0.1928 | 0.9044 | 0.0951 |
| $(X_2/Y) \times 100$ | 0.6697 | 0.3536 | 0.6624 | 0.2262 |
| $(X_3/Y) \times 100$ | 0.0856 | 0.0843 | 0.1346 | 0.0773 |
| $(X_4/Y) \times 100$ | 0.2534 | 0.1367 | 0.2756 | 0.1075 |
| $((X_1 + X_2)/Y) \times 100$ | 1.3459 | 0.5463 | 1.5668 | 0.3213 |
| $((X_3 + X_4)/Y) \times 100$ | 0.3390 | 0.2210 | 0.4102 | 0.1848 |
| $((X_1 + X_2 + X_3 + X_4)/Y) \times 100$ | 1.6849 | 0.7673 | 1.9770 | 0.5061 |
| Number of valid spots | 1632 | 1582 | 1577 | 1693 |
| Detection rate (%) | 63 | 61 | 61 | 65 |

[Table 4-2]

|  | Comparative Examples | | | | | | | |
|---|---|---|---|---|---|---|---|---|
|  | 1 | 2 | 3 | 4 | 5 | 6 | 7 | 8 |
| Peak area value of First peptide fragment ($X_1$) | 7534 | 23335 | 125531 | 1405 | 16063 | 1406 | 37705 | 33511 |
| Peak area value of Second peptide fragment ($X_2$) | 4536 | 2811195 | 5945 | 124326 | 17235 | 6523 | 118914 | 672270 |

(continued)

| | Comparative Examples | | | | | | | |
|---|---|---|---|---|---|---|---|---|
| | 1 | 2 | 3 | 4 | 5 | 6 | 7 | 8 |
| Peak area value of Third peptide fragment ($X_3$) | 5580 | 637579 | 37977 | 78293 | 2662 | 481 | 12520 | 107131 |
| Peak area value of Fourth peptide fragment ($X_4$) | 1050 | 564467 | 2922 | 22250 | 1197 | 2012 | 18566 | 464618 |
| Peak area value of Alkaline phosphatase (Y) | 268197 | 288388 | 245377 | 208272 | 232234 | 232042 | 272193 | 276191 |
| $(X_1/Y) \times 100$ | 2.8091 | 8.0915 | 51.1585 | 0.6746 | 6.9167 | 0.6061 | 13.8523 | 12.1333 |
| $(X_2/Y) \times 100$ | 1.6913 | 974.7951 | 2.4228 | 59.6940 | 7.4215 | 2.8110 | 43.6874 | 243.4081 |
| $(X_3/Y) \times 100$ | 2.0806 | 221.0835 | 15.4770 | 37.5917 | 1.1462 | 0.2073 | 4.5999 | 38.7888 |
| $(X_4/Y) \times 100$ | 0.3915 | 195.7316 | 1.1908 | 10.6831 | 0.5153 | 0.8669 | 6.8208 | 168.2237 |
| $((X_1 + X_2)/Y) \times 100$ | 4.5004 | 982.8866 | 53.5813 | 60.3686 | 14.3382 | 3.4171 | 57.5397 | 255.5414 |
| $((X_3 + X_4)/Y) \times 100$ | 2.4721 | 416.8151 | 16.6679 | 48.2748 | 1.6615 | 1.0742 | 11.4206 | 207.0126 |
| $((X_1 + X_2 + X_3 + X_4)/Y) \times 100$ | 6.9725 | 1399.7017 | 70.2492 | 108.6435 | 15.9997 | 4.4912 | 68.9604 | 462.5539 |
| Number of valid spots | 1442 | 1080 | 1259 | 1241 | 1211 | 1153 | 1245 | 1205 |
| Detection rate (%) | 56 | 42 | 49 | 48 | 47 | 45 | 48 | 47 |

As shown in Tables 4-1 and 4-2, when each of the nucleic acid samples prepared by using the alkaline phosphatase compositions of Comparative Examples 1 to 8 was used in the nucleic acid detection method, the number of valid spots was less than 1,500, while, when each of the nucleic acid samples prepared by using the alkaline phosphatase compositions of Examples 1 to 4 was used in the nucleic acid detection method, the number of valid spots was 1,500 or more. The detection rates in Comparative Examples 1 to 8 were different although the alkaline phosphatase specific activities of the alkaline phosphatase compositions were almost the same, while the detection rates in Examples 1 to 4 were almost the same and were higher than the detection rates in Comparative Examples 1 to 8.

As shown in Tables 4-1 and 4-2, the value of $((X_1 + X_2 + X_3 + X_4)/Y) \times 100$, which represents the content ratio of a total of the first to fourth peptide fragments to the alkaline phosphatase, was more than 4.4000 for each of the alkaline phosphatase compositions of Comparative Examples 1 to 8 (the minimum value was 4.4912 in the alkaline phosphatase composition of Comparative Example 6), while the value was 4.4000 or less for each of the alkaline phosphatase compositions of Examples 1 to 4. This is considered as one of the primary causes of the difference in the effect between Examples 1 to 4 and Comparative Examples 1 to 8.

As shown in Tables 4-1 and 4-2, the value of $((X_1 + X_2)/Y) \times 100$, which represents the content ratio of a total of the first and second peptide fragments to the alkaline phosphatase, was more than 3.4000 for each of the alkaline phosphatase compositions of Comparative Examples 1 to 8 (the minimum value was 3.4171 in the alkaline phosphatase composition of Comparative Example 6), while the value was 3.4000 or less for each of the alkaline phosphatase compositions of Examples 1 to 4. This is considered as one of the primary causes of difference in the effect mentioned above between Examples 1 to 4 and Comparative Examples 1 to 8.

As shown in Tables 4-1 and 4-2, the value of $((X_3 + X_4)/Y) \times 100$, which represents the content ratio of a total of the third and fourth peptide fragments to the alkaline phosphatase, was more than 1.0000 for each of the alkaline phosphatase compositions of Comparative Examples 1 to 8 (the minimum value was 1.0742 in the alkaline phosphatase composition of Comparative Example 6), while the value was 1.0000 or less for each of the alkaline phosphatase compositions of Examples 1 to 4. This is considered as one of the primary causes of the difference in the effect between Examples 1 to 4 and Comparative Examples 1 to 8.

As shown in Tables 4-1 and 4-2, the value of $(X_2/Y) \times 100$, which represents the content ratio of the second peptide fragment to the alkaline phosphatase, was more than 1.6000 for each of the alkaline phosphatase compositions of Comparative Examples 1 to 8 (the minimum value was 1.6913 in the alkaline phosphatase composition of Comparative Example 1), while the value was 1.6000 or less for each of the alkaline phosphatase compositions of Examples 1 to 4. This is considered as one of the primary causes of the difference in the effect between Examples 1 to 4 and Comparative Examples 1 to 8.

As shown in Tables 4-1 and 4-2, the value of $(X_3/Y) \times 100$, which represents the content ratio of the third peptide fragment to the alkaline phosphatase, was more than 0.2000 for each of the alkaline phosphatase compositions of Comparative Examples 1 to 8 (the minimum value was 0.2073 in the alkaline phosphatase composition of Comparative Example 6), while the value was 0.2000 or less for each of the alkaline phosphatase compositions of Examples 1 to 4. This is considered as one of the primary causes of the difference in the effect between Examples 1 to 4 and Comparative Examples 1 to 8.

As shown in Tables 4-1 and 4-2, the value of $(X_4/Y) \times 100$, which represents the content ratio of the fourth peptide fragment to the alkaline phosphatase, was more than 0.3500 for each of the alkaline phosphatase compositions of Comparative Examples 1 to 8 (the minimum value was 0.3915 in the alkaline phosphatase composition of Comparative Example 1), while the value was 0.3500 or less for each of the alkaline phosphatase compositions of Examples 1 to 4. This is considered as one of the primary causes of the difference in the effect between Examples 1 to 4 and Comparative Examples 1 to 8.

As shown in Tables 4-1 and 4-2, the value of $(X_1/Y) \times 100$, which represents the content ratio of the first peptide fragment to the alkaline phosphatase, was more than 1.0000 for each of the alkaline phosphatase compositions of Comparative Examples 1 to 3, 5, 7 and 8 (but 1.0000 or less for each of Comparative Examples 4 and 6), while the value was 1.0000 or less for each of the alkaline phosphatase compositions of Examples 1 to 4. This is considered as one of the secondary causes of the difference in the effect between Examples 1 to 4 and Comparative Examples 1 to 3, 5, 7 and 8.

**Claims**

1. A composition comprising:

   an alkaline phosphatase; and
   a peptide fragment group (A) composed of two or more peptide fragments, wherein each of the two or more peptide fragments consists of 5 to 50 consecutive amino acid residues selected from positions 501 to 578 of the amino acid sequence set forth in SEQ ID NO: 5,
   wherein a content ratio of the peptide fragment group (A) to the alkaline phosphatase satisfies the following formula (A):

$$(X_A/Y) \times 100 \leq 4.4000 \quad (A),$$

   wherein $X_A$ represents a peak area value of the peptide fragment group (A) calculated by an automatic integration method from an extracted ion chromatogram obtained by an LC-MS/MS analysis of the composition, and Y represents a peak area value of the alkaline phosphatase calculated by an automatic integration method from a chromatogram obtained by an LC-UV analysis of the composition.

2. A composition comprising:

an alkaline phosphatase; and
a peptide fragment group (B) composed of two or more peptide fragments, wherein each of the two or more peptide fragments consists of 13 to 50 consecutive amino acid residues selected from positions 501 to 578 of the amino acid sequence set forth in SEQ ID NO: 5 and comprises positions 516 to 528 of the amino acid sequence set forth in SEQ ID NO: 5,
wherein a content ratio of the peptide fragment group (B) to the alkaline phosphatase satisfies the following formula (B):

$$(X_B/Y) \times 100 \leq 3.4000 \qquad (B),$$

wherein $X_B$ represents a peak area value of the peptide fragment group (B) calculated by an automatic integration method from an extracted ion chromatogram obtained by an LC-MS/MS analysis of the composition, and Y represents a peak area value of the alkaline phosphatase calculated by an automatic integration method from a chromatogram obtained by an LC-UV analysis of the composition.

3. A composition comprising:

an alkaline phosphatase; and
a peptide fragment group (C) composed of two or more peptide fragments, wherein each of the two or more peptide fragments consists of 12 to 50 consecutive amino acid residues selected from positions 501 to 578 of the amino acid sequence set forth in SEQ ID NO: 5 and comprises positions 534 to 545 of the amino acid sequence set forth in SEQ ID NO: 5,
wherein a content ratio of the peptide fragment group (C) to the alkaline phosphatase satisfies the following formula (C):

$$(X_C/Y) \times 100 \leq 1.0000 \qquad (C),$$

wherein Xc represents a peak area value of the peptide fragment group (C) calculated by an automatic integration method from an extracted ion chromatogram obtained by an LC-MS/MS analysis of the composition, and Y represents a peak area value of the alkaline phosphatase calculated by an automatic integration method from a chromatogram obtained by an LC-UV analysis of the composition.

4. A composition comprising:

an alkaline phosphatase; and
a second peptide fragment consisting of the amino acid sequence set forth in SEQ ID NO: 2,
wherein a content ratio of the second peptide fragment to the alkaline phosphatase satisfies the following formula (2):

$$(X_2/Y) \times 100 \leq 1.6000 \qquad (2),$$

wherein $X_2$ represents a peak area value of the second peptide fragment calculated by an automatic integration method from an extracted ion chromatogram obtained by an LC-MS/MS analysis of the composition, and Y represents a peak area value of the alkaline phosphatase calculated by an automatic integration method from a chromatogram obtained by an LC-UV analysis of the composition.

5. The composition according to claim 4, wherein:

the composition further comprises a first peptide fragment consisting of the amino acid sequence set forth in SEQ ID NO: 1; and
a content ratio of the first peptide fragment to the alkaline phosphatase satisfies the following formula (1):

$$(X_1/Y) \times 100 \leq 1.0000 \qquad (1),$$

wherein $X_1$ represents a peak area value of the first peptide fragment calculated by an automatic integration method from an extracted ion chromatogram obtained by an LC-MS/MS analysis of the composition, and Y is the same as defined above.

6. The composition according to claim 4 or 5, wherein:

the composition further comprises a third peptide fragment consisting of the amino acid sequence set forth in SEQ ID NO: 3; and
a content ratio of the third peptide fragment to the alkaline phosphatase satisfies the following formula (3):

$$(X_3/Y) \times 100 \leq 0.2000 \qquad (3),$$

wherein $X_3$ represents a peak area value of the third peptide fragment calculated by an automatic integration method from an extracted ion chromatogram obtained by an LC-MS/MS analysis of the composition, and Y is the same as defined above.

7. The composition according to any one of claims 4 to 6, wherein:

the composition further comprises a fourth peptide fragment consisting of the amino acid sequence set forth in SEQ ID NO: 4; and
a content ratio of the fourth peptide fragment to the alkaline phosphatase satisfies the following formula (4):

$$(X_4/Y) \times 100 \leq 0.3500 \qquad (4),$$

wherein $X_4$ represents a peak area value of the fourth peptide fragment calculated by an automatic integration method from an extracted ion chromatogram obtained by an LC-MS/MS analysis of the composition, and Y is the same as defined above.

8. A composition comprising:

an alkaline phosphatase; and
a third peptide fragment consisting of the amino acid sequence set forth in SEQ ID NO: 3,
wherein a content ratio of the third peptide fragment to the alkaline phosphatase satisfies the following formula (3):

$$(X_3/Y) \times 100 \leq 0.2000 \qquad (3),$$

wherein $X_3$ represents a peak area value of the third peptide fragment calculated by an automatic integration method from an extracted ion chromatogram obtained by an LC-MS/MS analysis of the composition, and Y is the same as defined above.

9. The composition according to claim 8, wherein:

the composition further comprises a first peptide fragment consisting of the amino acid sequence set forth in SEQ ID NO: 1; and
a content ratio of the first peptide fragment to the alkaline phosphatase satisfies the following formula (1):

$$(X_1/Y) \times 100 \leq 1.0000 \qquad (1),$$

wherein $X_1$ represents a peak area value of the first peptide fragment calculated by an automatic integration method from an extracted ion chromatogram obtained by an LC-MS/MS analysis of the composition, and Y is the same as defined above.

10. The composition according to claim 8 or 9, wherein:

the composition further comprises a fourth peptide fragment consisting of the amino acid sequence set forth in SEQ ID NO: 4; and
a content ratio of the fourth peptide fragment to the alkaline phosphatase satisfies the following formula (4):

$$(X_4/Y) \times 100 \leq 0.3500 \qquad (4),$$

wherein $X_4$ represents a peak area value of the fourth peptide fragment calculated by an automatic integration method from an extracted ion chromatogram obtained by an LC-MS/MS analysis of the composition, and Y is the same as defined above.

11. A composition comprising:

an alkaline phosphatase; and
a fourth peptide fragment consisting of the amino acid sequence set forth in SEQ ID NO: 4,
wherein a content ratio of the fourth peptide fragment to the alkaline phosphatase satisfies the following formula (4):

$$(X_4/Y) \times 100 \leq 0.3500 \qquad (4),$$

wherein $X_4$ represents a peak area value of the fourth peptide fragment calculated by an automatic integration method from an extracted ion chromatogram obtained by an LC-MS/MS analysis of the composition, and Y is the same as defined above.

12. The composition according to claim 11, wherein:

the composition further comprises a first peptide fragment consisting of the amino acid sequence set forth in SEQ ID NO: 1; and
a content ratio of the first peptide fragment to the alkaline phosphatase satisfies the following formula (1):

$$(X_1/Y) \times 100 \leq 1.0000 \qquad (1),$$

wherein $X_1$ represents a peak area value of the first peptide fragment calculated by an automatic integration method from an extracted ion chromatogram obtained by an LC-MS/MS analysis of the composition, and Y is the same as defined above.

13. A method for producing a dephosphorylated nucleic acid, the method comprising the following steps of:

providing the composition according to any one of claims 1 to 12;
providing a nucleic acid; and
treating the nucleic acid with the composition to dephosphorylate the nucleic acid.

14. A method for producing a labeled nucleic acid, the method comprising the following steps of:

providing the composition according to any one of claims 1 to 12;
providing a nucleic acid;
providing a labeling substance;
treating the nucleic acid with the composition to dephosphorylate the nucleic acid; and
binding the labeling substance to the dephosphorylated nucleic acid.

FIG. 1

Fig. 2

Fig. 3

Fig. 4

Fig. 5

Fig. 6

Fig. 7

Fig. 8

Fig. 9

## INTERNATIONAL SEARCH REPORT

| International application No. |
| --- |
| PCT/JP2019/037520 |

**A. CLASSIFICATION OF SUBJECT MATTER**
Int.Cl. C12N9/16(2006.01)i, C12N15/10(2006.01)i, C12N15/55(2006.01)i, C12Q1/6876(2018.01)i

According to International Patent Classification (IPC) or to both national classification and IPC

**B. FIELDS SEARCHED**

Minimum documentation searched (classification system followed by classification symbols)
Int.Cl. C12N9/16, C12N15/10, C12N15/55, C12Q1/6876

Documentation searched other than minimum documentation to the extent that such documents are included in the fields searched
Published examined utility model applications of Japan     1922–1996
Published unexamined utility model applications of Japan   1971–2019
Registered utility model specifications of Japan           1996–2019
Published registered utility model applications of Japan   1994–2019

Electronic data base consulted during the international search (name of data base and, where practicable, search terms used)
CAplus/REGISTRY/MEDLINE/EMBASE/BIOSIS (STN)
JSTPlus/JMEDPlus/JST7580 (JDreamIII)
GenBank/EMBL/DDBJ/GeneSeq, UniProt/GeneSeq, SwissProt/GeneSeq

**C. DOCUMENTS CONSIDERED TO BE RELEVANT**

| Category* | Citation of document, with indication, where appropriate, of the relevant passages | Relevant to claim No. |
| --- | --- | --- |
| X | POZIDIS, C., BOURIOTIS, V., "Preparation of high purity alkaline phosphatase from calf intestine | 1–14 |
| Y | using dye-ligand chromatography", Bioseparation, 1995, vol. 5, no. 2, pp. 89-93, abstract, introduction | 1–14 |
| Y | US 2008/0098491 A1 (ZOLTAN LABORATORIES LLC) 24 April 2008, example 1 (Family: none) | 1–14 |

☒ Further documents are listed in the continuation of Box C.     ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 10 December 2019 (10.12.2019) | 17 December 2019 (17.12.2019) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
|---|
| PCT/JP2019/037520 |

C (Continuation). DOCUMENTS CONSIDERED TO BE RELEVANT

| Category* | Citation of document, with indication, where appropriate, of the relevant passages | Relevant to claim No. |
|---|---|---|
| Y | US 2007/0148140 A1 (ZOLTAN LABORATORIES LLC) 28 June 2007, example 1 & WO 2007/081654 A2 & EP 1988917 A1 | 1-14 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H10262674 A
- WO 2012115023 A
- US 5705610 A, Ronald
- US 6142266 A, Michel
- US 7037659 A, Francesco
- JP H10503841 A
- JP 3922454 B, Hirota